# EUROPEAN PATENT SPECIFICATION

(11) **EP 3 099 821 B1**
(45) Date of publication and mention of the grant of the patent: **11.08.2021**
(21) Application number: 15740989.7
(22) Date of filing: 26.01.2015
(51) Int. Cl.: G01N 33/574, C12Q 1/6886

(54) **CIRCULATING TUMOR CELL DIAGNOSTICS FOR DETECTION OF NEUROENDOCRINE PROSTATE CANCER (NEPC)**
DIAGNOSTIKA FÜR ZIRKULIERENDE TUMORZELLEN ZUM NACHWEIS VON NEUROENDOKRINEM PROSTATAKREBS (NEPC)
DIAGNOSTIC DE CELLULES TUMORALES CIRCULANTES POUR DÉTECTER LE CANCER NEUROENDOCRINE DE LA PROSTATE (CNEP)

(30) Priority: 27.01.2014 US 201461932172 P
(43) Date of publication of application: 07.12.2016
(73) Proprietor: Epic Sciences, Inc., San Diego, CA 92121 (US)
(72) Inventor: DITTAMORE, Ryan, San Diego, CA 92121 (US)
(74) Representative: Jones Day
(86) International application number: PCT/US2015/012867
(87) International publication number: WO 2015/112960

(56) References cited:
- US-A1- 2010 048 709
- US-A1- 2012 276 555
- FANNY CHAN ET AL: "Dramatically Elevated Circulating Tumor Cell Numbers in a Patient With Small Cel l Neuroendocrine Carcinoma of the Prostate", ARCH PATHOL LAB MED-VOL, vol. 134, 1 January 2013 (2013-01-01), pages 120-123, XP055357379,
- H Beltran ET AL: "Challenges in Recognizing Treatment-Related Neuroendocrine Prostate Cancer", , 20 December 2012 (2012-12-20), XP055382244, Retrieved from the Internet: URL:http://ascopubs.org/doi/pdfdirect/10.1 200/JCO.2011.41.5166 [retrieved on 2017-06-16]
- H. BELTRAN ET AL: "The Initial Detection and Partial Characterization of Circulating Tumor Cells in Neuroendocrine Prostate Cancer", CLINICAL CANCER RESEARCH, vol. 22, no. 6, 15 March 2016 (2016-03-15) , pages 1510-1519, XP055382275, US ISSN: 1078-0432, DOI: 10.1158/1078-0432.CCR-15-0137
- CHAN ET AL.: 'Dramatically Elevated Circulating Tumor Cell Numbers in a Patient With Small Cell Neuroendocrine Carcinoma of the Prostate' ARCH PATHOL LAB MED. vol. 134, 2010, pages 120 - 123, XP055357379
- PARK ET AL.: 'Morphological Differences between Circulating Tumor Cells from Prostate Cancer Patients and Cultured Prostate Cancer Cells' PLOS ONE vol. 9, no. 1, 08 January 2014, pages 1 - 7, XP055215611
- Anonymous: "Circulating tumor cell", Wikipedia, 13 January 2015 (2015-01-13), XP055472147, Retrieved from the Internet: URL:https://en.wikipedia.org/w/index.php?t itle=Circulating_tumor_cell&oldid=64223529 5 [retrieved on 2018-05-03]
- RACILA E ET AL: "Detection and charcterization of carcinoma cells in the blood", PROCEEDINGS OF THE NATIONAL ACADEMY OF SCIENCES, NATIONAL ACADEMY OF SCIENCES, US, vol. 95, 1 April 1998 (1998-04-01), pages 4589-4594, XP002132943, ISSN: 0027-8424, DOI: 10.1073/PNAS.95.8.4589
- PETER SIDAWAY: "Non-traditional CTCs indicate prognosis : Urological cancer", NATURE REVIEWS CLINICAL ONCOLOGY, vol. 13, no. 10, 7 September 2016 (2016-09-07), pages 592-592, XP055472116, NY, US ISSN: 1759-4774, DOI: 10.1038/nrclinonc.2016.146
- FERRALDESCHI R ET AL: "CK- and small nuclear size circulating tumor cell (CTCs) phenotypes in metastatic castration-resistant prostate cancer (mCRPC)", INTERNET CITATION, 1 January 2014 (2014-01-01), page ABST.209, XP002765660, Retrieved from the Internet: URL:http://meetinglibrary.asco.org/print/1 447426 [retrieved on 2016-01-06]

## Description

The present disclosure relates generally to methods for identification and molecular characterization of the CTCs associated with neuroendocrine prostate cancer (NEPC).

### BACKGROUND

Prostate cancer is the most commonly diagnosed solid organ malignancy in the United States (US) and remains the second leading cause of cancer deaths among American men. In 2014 alone, the projected incidence of prostate cancer is 233,000 cases with deaths occurring in 29,480 men, making metastatic prostate cancer therapy truly an unmet medical need. Siegel et al., 2014. CA Cancer J Clin. 2014;64(1):9-29. Epidemiological studies from Europe show comparable data with an estimated incidence of 416700 new cases in 2012, representing 22.8% of cancer diagnoses in men. In total, 92200 PC-specific deaths are expected, making it one of the three cancers men are most likely to die from, with a mortality rate of 9.5%

With the recent advent of exponential growth of novel agents tested and approved for the treatment of patients with metastatic castration-resistant prostate cancer (mCRPC), the advanced stage of prostate cancer that typically accounts for prostate cancer deaths, issues regarding the optimal sequencing or combination of these agents have arisen. The sequence of administration of medications with distinct mechanisms of action, toxicities and efficacies, will have a critical role in disease outcomes. Several guidelines exist that help direct clinicians as to the best sequencing approach and most would evaluate presence or lack of symptoms, performance status, as well as burden of disease to help determine the best sequencing for these agents. Mohler et al., 2014, J Natl Compr Canc Netw. 2013;11(12):1471-1479; Cookson et al. , 2013, J Urol. 2013;190(2):429-438. Currently, approved treatments consist of the taxane class of cytotoxic drugs and androgen-targeted therapies. The challenge for clinicians is to decide the best sequence for administering these therapies to provide the greatest benefit to patients. However, therapy failure remains a significant challenge based on heterogenous responses to therapies across patients and in light of cross-resistance from each agent. Mezynski et al. , Ann Oncol. 2012;23(11):2943-2947;. Noonan et al., Ann Oncol. 2013;24(7):1802-1807; Pezaro et al., Eur Urol. 2014, 66(3): 459-465.

Neuroendocrine prostate cancer (NEPC) is an aggressive androgen independent variant of prostate cancer that most commonly arises in later stages of mCRPC as a mechanism of treatment resistance. Androgen receptor (AR) expression is typically low or absent in NEPC and the genes Aurora kinase A (AURKA) and N-Myc (MYCN) are frequently amplified. There are no reliable biomarkers to identify patients that are transforming to NEPC and incidence of CTCs in these patients is unknown. Detection of NEPC has clinical implications as these patients would not be expected to respond to currently approved potent AR targeted therapies for CRPC.

Circulating tumor cells (CTCs) represent a significant advance in cancer diagnosis made even more attractive by their non-invasive measurement. Cristofanilli et al., N Engl J Med 2004, 351:781-91. CTCs released from either a primary tumor or its metastatic sites hold important information about the biology of the tumor. Historically, the extremely low levels of CTCs in the bloodstream combined with their unknown phenotype has significantly impeded their detection and limited their clinical utility. A variety of technologies have recently emerged for detection, isolation and characterization of CTCs in order to utilize their information. CTCs have the potential to provide a non-invasive means of assessing progressive cancers in real time during therapy, and further, to help direct therapy by monitoring phenotypic physiological and genetic changes that occur in response to therapy. In most advanced prostate cancer patients, the primary tumor has been removed, and CTCs are expected to consist of cells shed from metastases, providing a "liquid biopsy." While CTCs are traditionally defined as EpCAM/cytokeratin positive (CK+) cells, CD45-, and morphologically distinct, recent evidence suggests that other populations of CTC candidates exist including cells that are EpCAM/cytokeratin negative (CK-) or cells smaller in size than traditional CTCs. These findings regarding the heterogeneity of the CTC population, suggest that enrichment-free CTC platforms are favorable over positive selection techniques that isolate CTCs based on size, density, or EpCAM positivity that are prone to miss important CTC subpopulations.

Chan *et al.* discloses the detection of CTCs in whole blood of a patient with neuroendocrine small cell prostate cancer (Chan et al. Arch Pathol Lab Med 2010, 134:120-123).

A need exists to develop accurate and non-invasive methods for identification of patients with de novo or treatment induced NEPC who will cease to respond to hormone targeted therapy so as to reduce morbidity and enable early exploration of alternative therapy approaches. The present invention addresses this need by providing a multivariate biomarker panel for detection of NEPC based on a robust CTC detection and characterization platform that enables the phenotypic characterization of CTCs. Related advantages are provided as well.

### SUMMARY

The present disclosure generally relates to methods for diagnosing NEPC in a patient afflicted with prostate cancer.

The present invention provides a method for detecting NEPC in a patient afflicted with prostate cancer comprising (a) performing a direct analysis comprising immunofluorescent staining and morphological characterization of nucleated cells in a blood sample obtained from the patient to detect circulating tumor cells (CTC), wherein the direct analysis means the detection of CTCs in the context of all surrounding nucleated cells present in the sample as opposed to after enrichment of the sample for CTCs prior to detection; and (b) determining presence or absence of a CTC subpopulation associated with NEPC comprising detecting a measurable feature of each biomarker characterizing the CTC subpopulation associated with NEPC in a panel of morphological and protein biomarkers, wherein the presence of the CTC subpopulation associated with NEPC is indicative of NEPC, and whrerein the biomarkers for the CTC subpopulation associated with NEPC comprise small size absence of Androgen Receptor (AR-), absence of cytokeratin (CK-) expression and presence of nucleoli (nucleoli+). In additional embodiments, the methods of the invention further comprise molecular analysis of the CTCs.

Described is a method for detecting atypical CRPC in a patient afflicted with prostate cancer comprising (a) performing a direct analysis comprising immunofluorescent staining and morphological characterization of nucleated cells in a blood sample obtained from the patient to detect circulating tumor cells (CTC), and (b) determining presence or absence of a CTC subpopulation associated with atypical CRPC comprising detecting a measurable feature of a panel of morphological and protein biomarkers, wherein the presence of the CTC subpopulation associated with atypical CRPC is indicative of atypical CRPC.

Described is further a method for detecting transformation of adenocarcinoma into NEPC in a subject afflicted with prostate cancer comprising (a) performing a direct analysis comprising immunofluorescent staining and morphological characteristization of nucleated cells in a blood sample obtained from the patient to detect circulating tumor cells (CTC); (b) determining presence or absence of a CTC subpopulation associated with NEPC comprising detecting a measurable feature of each biomarker in a panel of morphological and protein biomarkers, wherein the presence of the CTC subpopulation associated with NEPC is indicative of NEPC, and (c) repeating steps (a) and (b), wherein emergence of the presence of the CTC population associated with NEPC indicates transformation of adenocarcinoma into NEPC. In some cases, the subject is undergoing hormone treatment. The emergence of the CTC population associated with NEPC may predict resistance to hormone treatment, and inform a subsequent decision to discontinue hormone treatment and/or to initiate cytotoxic therapy.

### BRIEF DESCRIPTION OF THE DRAWINGS

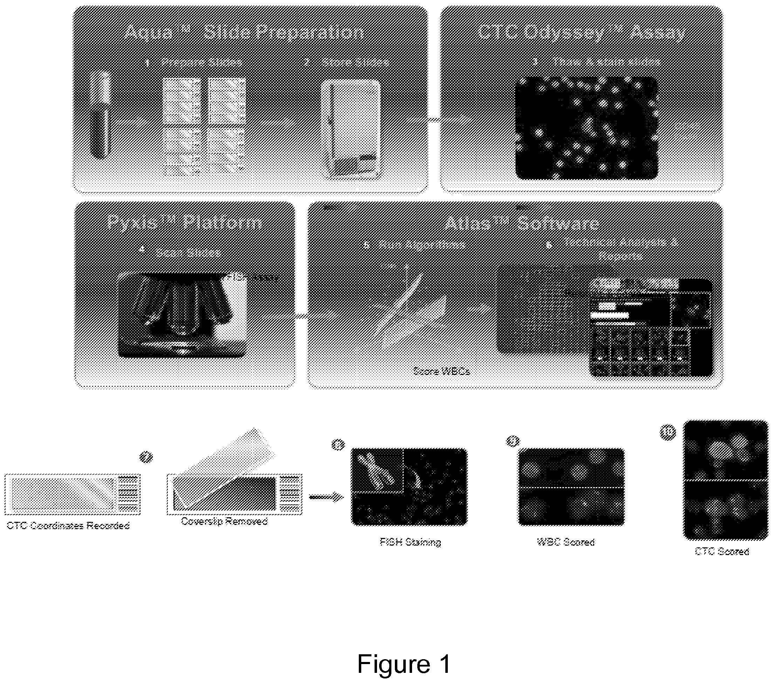

Figure 1 shows methods used in performing the embodiments exemplified herein. Figure 1 shows a schematic of a representative CTC collection and detection process: (1) nucleated cells from blood sample placed onto slides; (2) slides stored in -80°C biorepository; (3) slides stained with CK, CD45, DAPI and AR; (4) slides scanned; (5) multi-parametric digital pathology algorithms run; (6) software and human reader confirmation of CTCs and quantitation of biomarker expression; (7) for FISH, coordinates are recorded and coverslip removed; (8) FISH assay is run; (9) regional WBCs are scored to assess normal; and (10) CTCs relocated and scored.

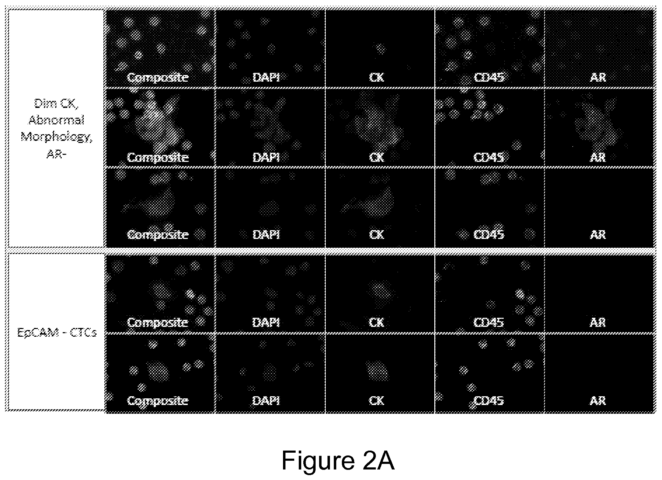

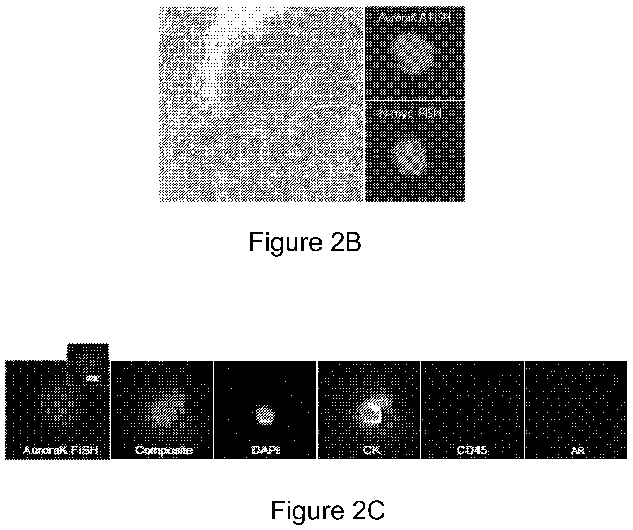

Figures 2A, 2B, and 2C show identification of novel CTC subpopulations in NEPC and AuroraK amplification seen in tissue and CTC samples. Figure 2A shows low CK CTCs with cancer related morphology are seen which have no AR and no EpCAM expression. CTCs from NEPC patients also exhibit unique spindle-like morphology. Figure 2B shows AuroraK amplification seen in tissue and CTC samples. Samples from Patient #6 were evaluated. Figure 2B shows castration resistant tumor tissue, where hematoxylin-eosin (H&E) shows poorly differentiated carcinoma, and Aurora kinase A and N-myc amplified by FISH. Figure 2C shows patient matched circulating tumor cells (CK+, CD45-, AR-), and Aurora kinase A also amplified by FISH.

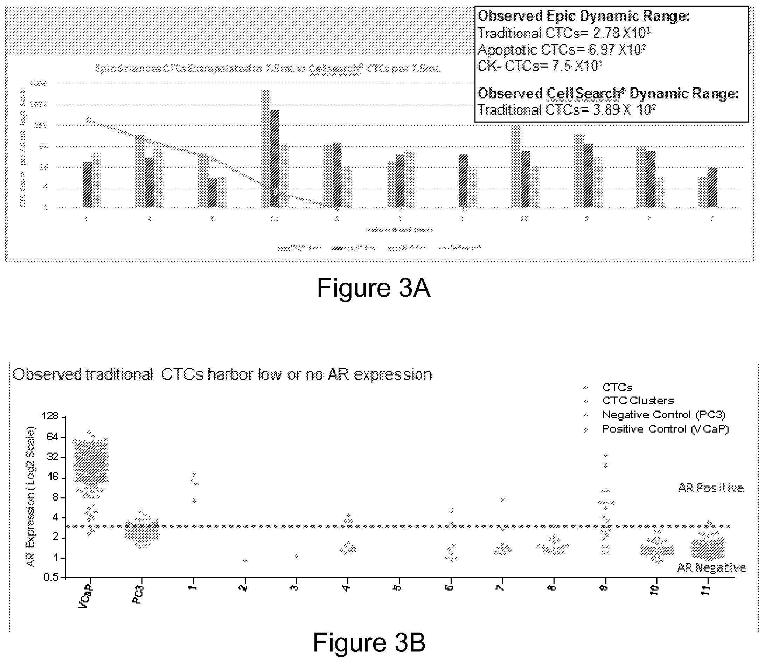

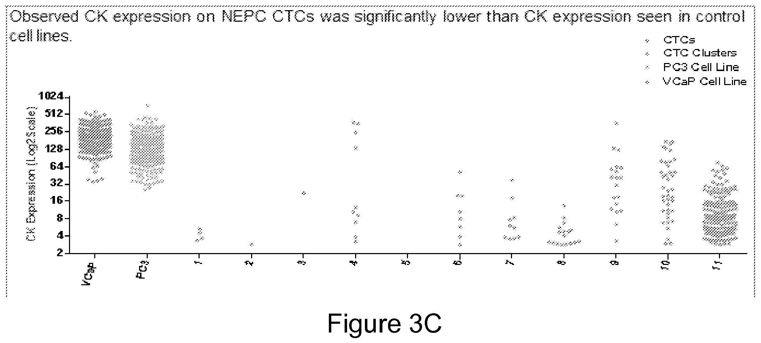

Figures 3A, 3B, and 3C show sensitivity of Epic CTC platform vs. CellSearch®, AR expression and distribution of CTCs, and CK expression and distribution of CTCs. Figure 3A shows matched blood samples were processed utilizing CellSearch® and Epic Sciences CTC platform. Epic CTCs were measured from 1 mL and extrapolated to 7.5 ml of blood. Figure 3B shows AR expression per CTC or CTC cluster plotted for each patient. Figure 3C shows CK expression per CTC or CTC cluster plotted for each patient.

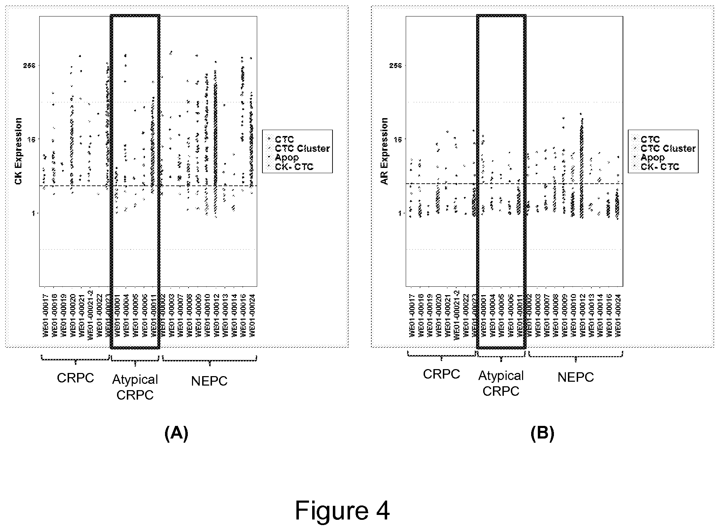

Figures 4A and 4B show CK Expression (Figure 4A) and AR Expression (Figure 4B) of CTCs from each patient sample organized by their clinical diagnosis.

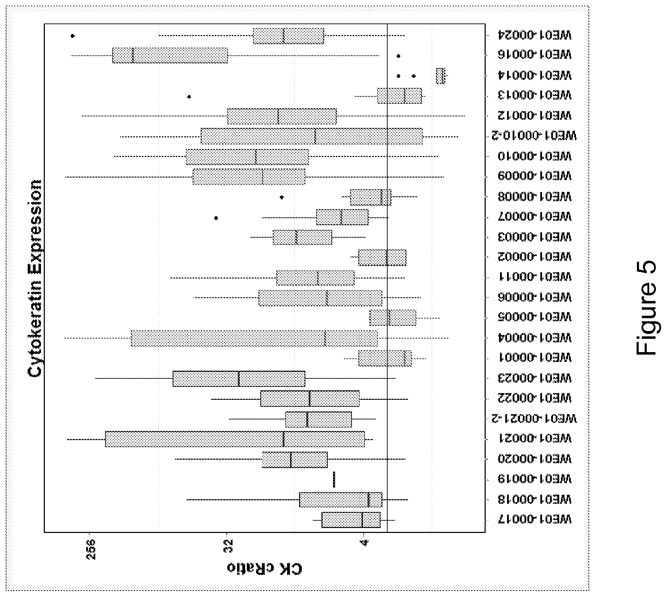

Figure 5 shows a boxplot of CK expression for patients diagnosed as CRPC (blue: WE01-00017, WE01-00018, WE01-00019, WE01-00020, WE01-00021, WE01-00021-2, WE01-00022, WE01-00023), atypical CRPC (green: WE01-00001, WE01-00004, WE01-00005, WE01-00006, WE01-00011), and NEPC (red: WE01-00002, WE01-00003, WE01-00007, WE01-00008, WE01-00009, WE01-00010, WE01-00010-2, WE01-00012, WE01-00013, WE01-00014, WE01-00016, WE01-00024). CTCs with a CK cRatio above the horizontal black line at y =2.8 are positive for Cytokeratin Expression. Boxes represent interquartile range.

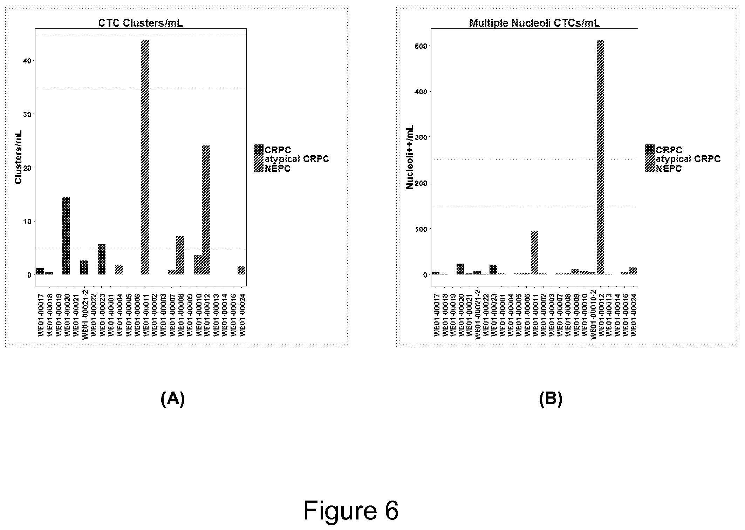

Figures 6A and 6B shows bar charts comparing the emergence of CTC clusters (Figure 6A, left) and CTC with multiple prominent nucleoli (Figure 6B, right) in NEPC (red, right bars), CRPC (blue, left bars), and atypical CRPC (green, middle bars) cohorts.

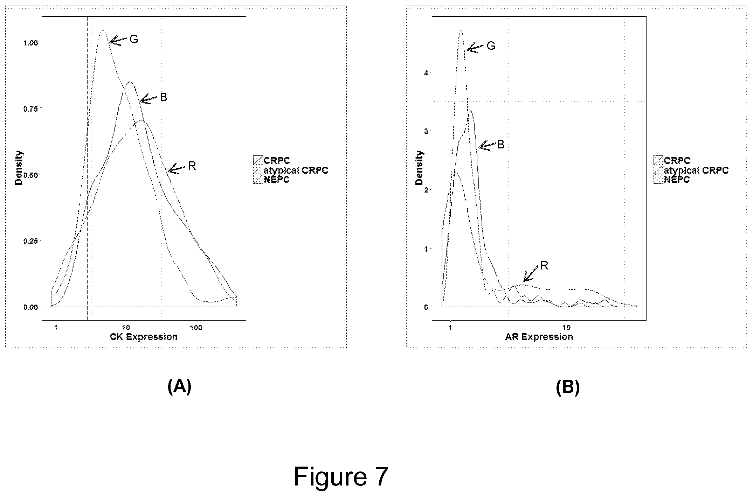

Figures 7A and 7B show Kernel density estimate (KDE) curves of Cytokeratin Expression (Figure 7A, left) and Androgen Receptor Expression (Figure 7B, right) for CTCs aggregated from all NEPC (red, R), CRPC (blue, B), and atypical CRPC (green, G) patient samples.

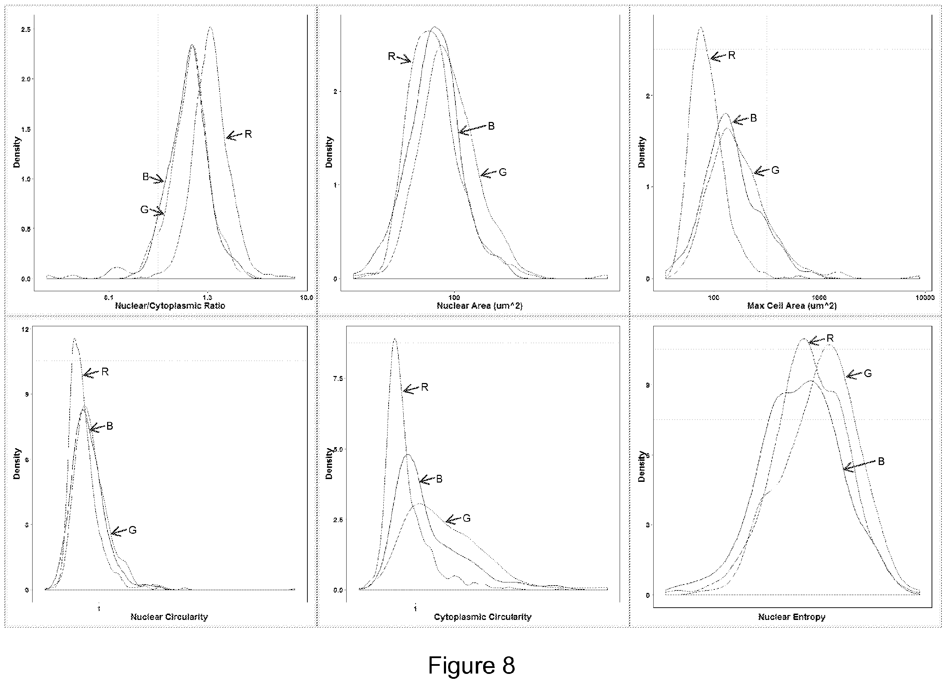

Figure 8 shows Kernel Density Estimate (KDE) curves for CTCs aggregated by patient clinical diagnosis: NEPC (red, R), CRPC (blue, B), and atypical CRPC (green, G).

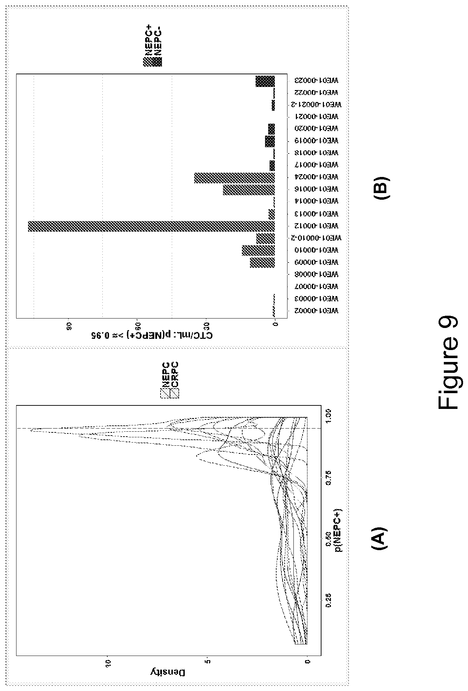

Figure 9A shows Kernel Density Estimate (KDE) curves of the classifier output are plotted for each patient sample colored by their diagnosis: NEPC (red) and CRPC (blue). Note the peak in densities near the far right of the curve corresponding to high probability of NEPC class membership for patient samples diagnosed with NEPC. Figure 9B shows a bar chart of the number of CTCs/mL that have an estimated probability of class membership to NEPC+ (left bars) greater than or equal to 0.95.

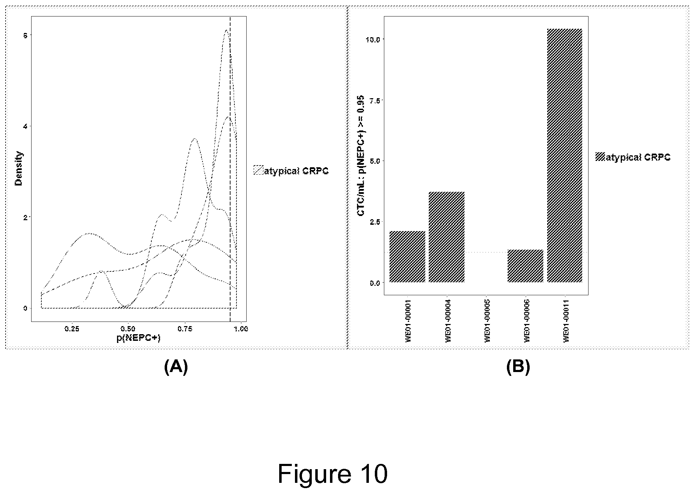

Figure 10A shows NEPC and CRPC patient samples were used to train a classifier, for which the atypical CRPC patient samples were analyzed as the test set. Kernel Density Estimate (KDE) curves of the classifier output are plotted for each atypical CRPC patient. Note the peak in densities near the far right of the curve corresponding to high probability of NEPC class membership. Figure 10B shows bar charts of the number of CTCs/mL that have an estimated probability of class membership to NEPC+ greater than or equal to 0.95.

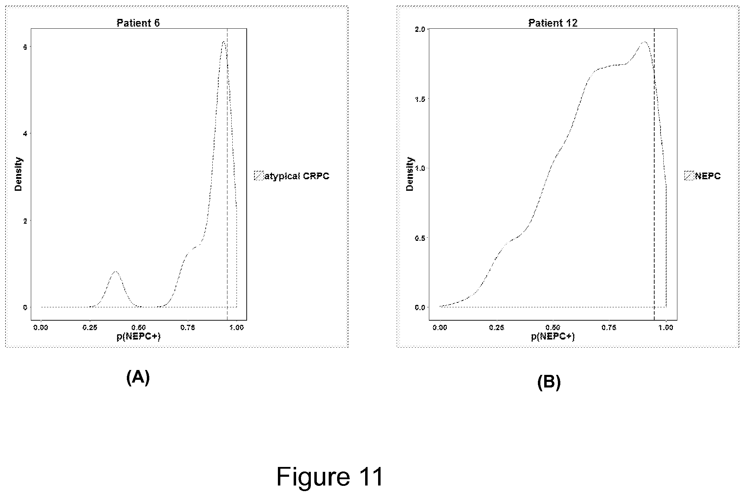

Figures 11A and 11B show NEPC probability class memberships for Patients 6 and 12. Figure 11A shows Patient 6 distribution obtained from Figure 10. Figure 11B shows Patient 12 distribution obtained from Figure 9.

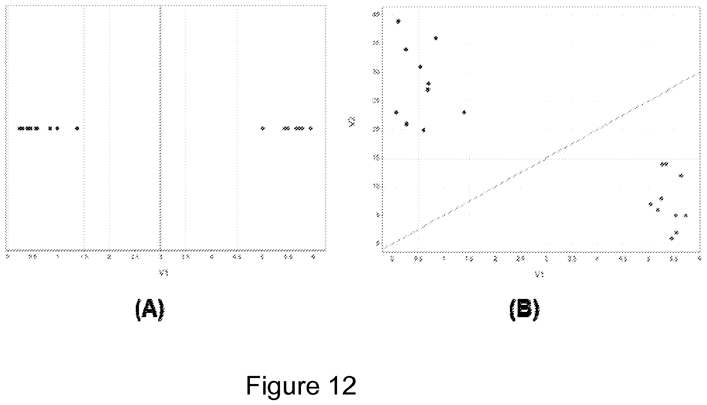

Figures 12A and 12B show examples of a decision boundary that separate class A (red, dots right of line) from class B (blue, dots left of line) in 1 dimension (Figure 12A) and 2 dimensions (Figure 12B). In three dimensions, the decision boundary would be a plane. In high dimensional space, this becomes difficult to visualize.

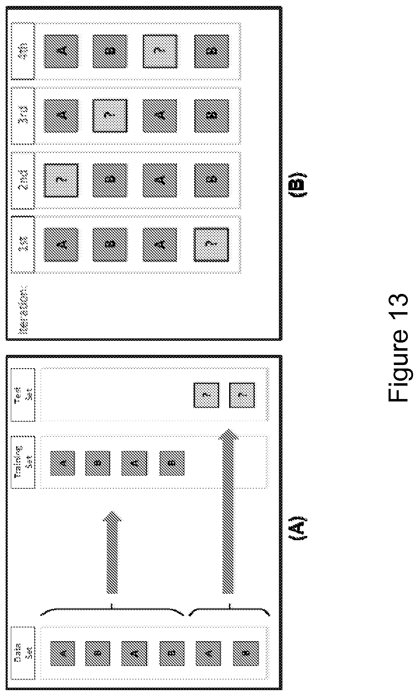

Figures 13A and 13B show a schematic of the supervised learning process (Figure 13A) and leave one out cross validation (Figure 13B). During each iteration, a dataset of examples labeled with their class membership (class A, class B) is partitioned into a training set (blue) and a test set (orange, shown with "?").

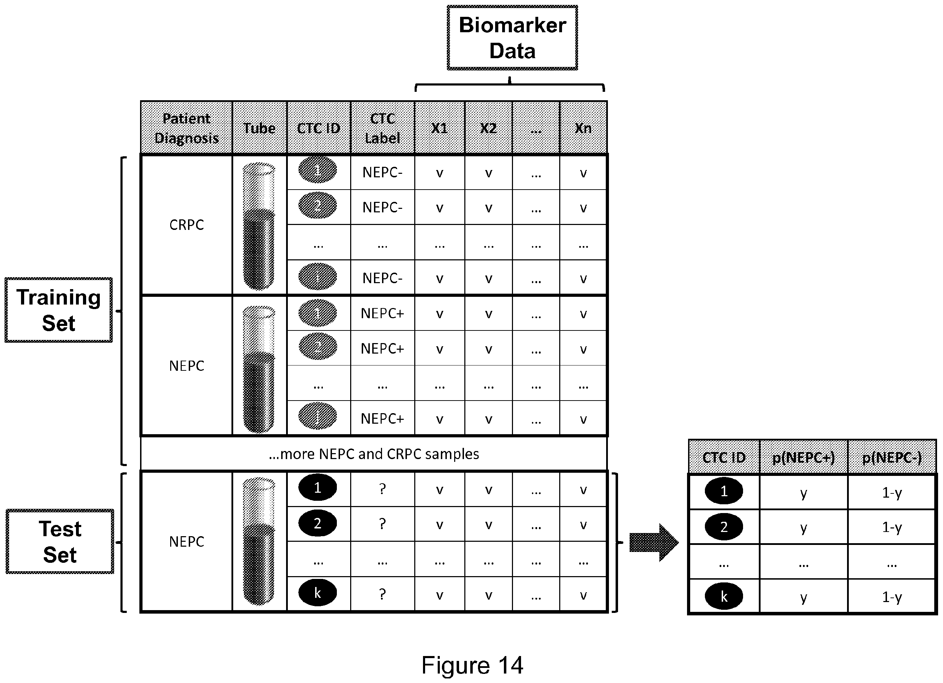

Figure 14 demonstrates single iteration of Leave-One-Out Cross-Validation performed at the blood sample level. CTCs from every other sample are partitioned as the Training Set, and presented to the classifier as labeled examples. CTCs from the sample held-out as the test set are then analyzed by the trained classifier, which provides an estimated probability of class membership to NEPC+ or NEPC- for each CTC (right).

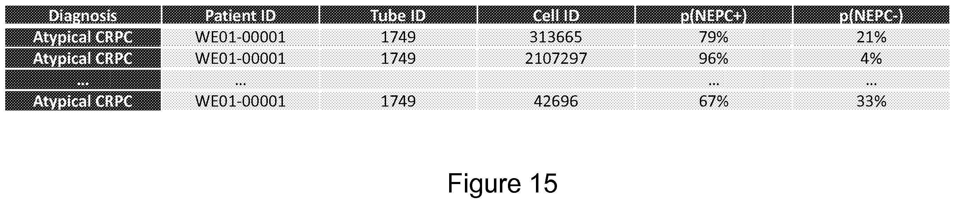

Figure 15 shows an example of results from a single tube of blood. The two columns on the right are the output from classification for each CTC: p(NEPC+) being the probability of the CTC belonging to NEPC, and p(NEPC-) being the probability of the CTC belonging to CRPC.

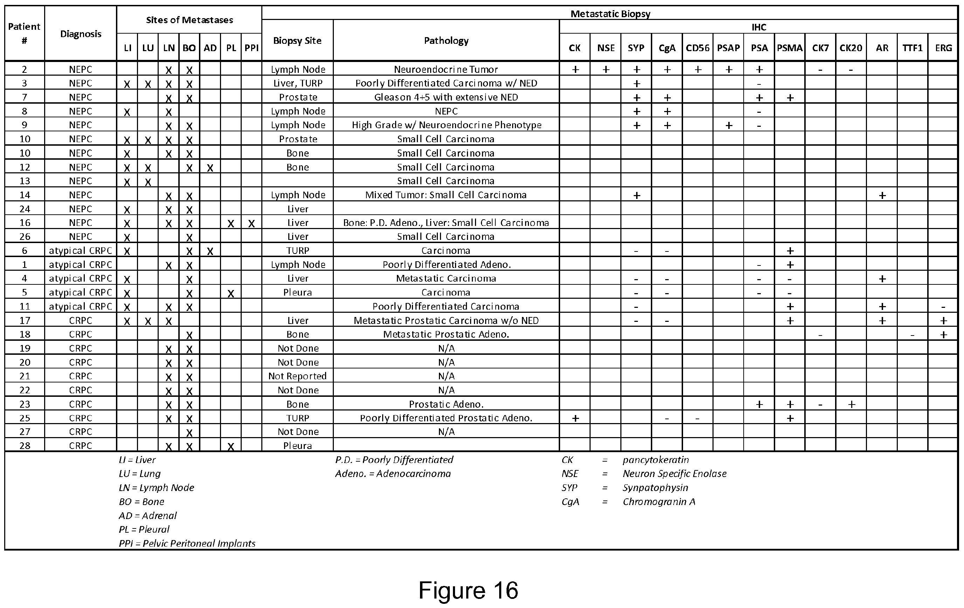

Figure 16 shows a table that describes the clinical data derived from each patient sample including: diagnosis, site of metastasis, biopsy site, pathological analysis and IHC results for common PCa markers.

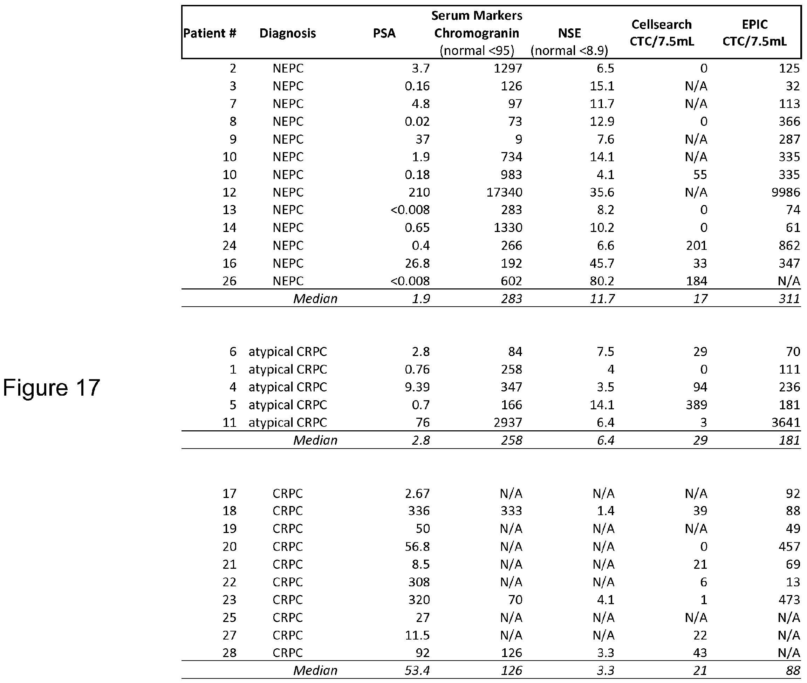

Figure 17 shows a table listing the observed data from the analysis for common serum PCa markers including PSA, Chromogranin, NSE and CTC counts for NEPC, atypical CRPC and CRPC patients individually.

### DETAILED DESCRIPTION

The present disclosure is based, in part, on the unexpected discovery that phenotypic characterization of CTCs obtained from prostate cancer patients can generate a diagnostic biomarker signature that can detect NEPC. NEPC is a hormone-refractory late manifestation of prostate cancer and represents about 25% of late-stage disease. NEPC has a poor prognosis, with most patients surviving for less than 1 year after diagnosis. While NEPC is most commonly the result of treatment with hormone therapy, known as treatment-related NEPC, or t-NEPC, in rare instances patients are diagnosed with *de novo* NEPC. The methods of the present invention encompass both treatment-related and *de novo* NEPC.

Despite the proven success of hormonal therapy for prostate cancer using chemical or surgical castration, most patients eventually will progress to a phase of the disease that is metastatic and shows resistance to further hormonal manipulation. This has been termed metastatic castrate-resistant prostate cancer (mCRPC). Despite castrate levels of androgens, the androgen receptor (AR) remains active and continues to drive prostate cancer progression. This understanding has led to the development of anti-androgen hormonal therapy drugs such as, for example, Zytiga (arbiterone, blocks androgen production) and Xtandi (enzalutamide, an AR inhibitor), which are beneficial in extending lives of adenocarcinoma patients. In a small percentage of patients, treatment with hormone therapy transforms adenocarcinoma into NEPC, which is not dependent upon androgen or androgen receptor. As a result, anti-androgen hormonal therapy drugs that are beneficial for mCRPC patients, do not confer a clinical benefit on patients with NEPC. Significantly, the methods disclosed herein enable detection of NEPC in a patient afflicted with prostate cancer and make it possible to distinguish between different patient groups in the resistance setting in order to tailor subsequent treatment more precisely and effectively. The methods may allow for resistance monitoring of a prostate cancer patients by enabling detection of an emergence of NEPC in a patient afflicted with prostate cancer.

Circulating tumor cells (CTCs) provide the potential for non-invasive, real-time molecular characterization of cancer in patients with metastatic disease. Yap et al., (2014) Clin Cancer Res 20: 2553-2568; Parkinson et al. (2012) J Transl Med 10: 138; Polzer et al. (2014) EMBO Mol Med 6: 1371-1386; Krebs et al. (2014) Nat Rev Clin Oncol 11: 129-144. To date, the only FDA-cleared test for CTC detection is the CellSearchⓇ technology, based on immunomagnetic enrichment of CTCs expressing epithelial cell adhesion molecular (EpCAM). Allard et al. (2004) Clin Cancer Res 10: 6897-6904; Cristofanilli et al. (2004) N Engl J Med 351: 781-791; Miller et al. (2010) J Oncol 2010: 617421. CellSearch® CTCs are traditionally defined as EpCAM/cytokeratin positive (CK+) cells, CD45-, and are morphologically distinct. The CellSearchⓇ technology has demonstrated clinical utility in metastatic prostate, breast, and colorectal cancer for prognosis in stage IV cancer (Miller et al. (2010) J Oncol 2010: 617421), but lacks sensitivity in many metastatic prostate cancer patients and is unable to characterize CTCs for morphologic, protein or genomic features. Several other strategies have recently been developed to capture CTCs in the blood, most of which include enrichment and/or other physical selection modalities (Nagrath et al. (2007) Nature 450: 1235-1239; Stott et al. (2010) Sci Transl Med 2: 25ra23; Vona et al. (2000) Am J Pathol 156: 57-63; Chinen et al. (2013) J Thorac Dis 5: 593-599), some of which have improved the sensitivity of CTC detection. Yap et al., (2014) Clin Cancer Res 20: 2553-2568; Ozkumur et al. (2013) Sci Transl Med 5: 179ra147. However, enrichment-based technologies make assumptions about the physical and molecular nature of CTCs that might reflect a limited spectrum of malignant cells in circulation. There is mounting evidence that non-traditional populations of CTC also exist including cells that are EpCAM/cytokeratin negative (CK-) (Yu et al. (2013) Science 339: 580-584) and/or cells smaller in size than traditional CTCs, some even smaller than neighboring white blood cells. Ozkumur et al. (2013) Sci Transl Med 5: 179ra147; Phillips et al. (2014) Am J Physiol Cell Physiol 306: C80-88. Because CTC enrichment techniques may miss these subpopulations, we aimed to molecularly characterize all CTCs from patients with NEPC utilizing the Epic Sciences platform which performs no physical selection, and to correlate the results with patient-matched tumor biopsy clinical, molecular, and pathologic features. The Epic Sciences technology described herein identifies CTCs through a multi-parametric digital pathology identification process which identifies abnormal cells among the normal white blood cells utilizing protein expression and morphology filters. Hsieh et al. (2006) Biosens Bioelectron 21: 1893-1899; Marrinucci et al. (2007) Hum Pathol 38: 514-519; Marrinucci et al. (2012) Phys Biol 9: 016003. This technique has demonstrated the ability to identify CTCs, apoptotic CTCs, CK- CTCs, and CTC clusters Marrinucci et al. (2012) Phys Biol 9: 016003.

In one aspect, the present invention provides a method for detecting NEPC in a patient afflicted with prostate cancer comprising (a) performing a direct analysis comprising immunofluorescent staining and morphological characterization of nucleated cells in a blood sample obtained from the patient to detect circulating tumor cells (CTC), wherein the direct analysis means the detection of CTCs in the context of all surrounding nucleated cells present in the sample as opposed to after enrichment of the sample for CTCs prior to detection; and (b) determining presence or absence of a CTC subpopulation associated with NEPC comprising detecting a measurable feature of a panel of morphological and protein biomarkers, wherein the presence of the CTC subpopulation associated with NEPC is indicative of NEPC, and wherein biomarkers observed in the CTC subpopulation associated with NEPC comprise small size, absence of Androgen Receptor (AR), absence of cytokeratin (CK-) expression and presence of nucleoli (nucleoli⁺). Biomarkers observed in the CTC subpopulation associated with NEPC may further comprise frequent clusters. In additional embodiments, the methods of the invention further comprise molecular analysis of the CTCs.

Table 1 lists morphological biomarkers useful in practicing the methods disclosed herein. Accordingly, die methods disclosed herein can comprise one or more of the morphological biomarkers set forth in Table 1.

**Table 1. Morphological Biomarkers**

| | Morphological Biomarker Category | Morphological Biomarker | | Measured Feature |
|---|---|---|---|---|
| 1 | CK Expression | CK cRatio | Numeric | |
| 2 | | ck_bin | Categorical | CK-, CK+, CK++ |
| 3 | AR Expression | AR Expression | Numeric | |
| 4 | | ar_bin | Categorical | AR-, AR+ |
| 5 | CTC Clustering | cluster_bin | Categorical | Binary (1 if CTC Cluster, 0 if not) |
| 6 | Nuclear Size | Nuclear Area | Numeric | |
| 7 | | Nuclear Convex Area | Numeric | |
| 8 | | Nuclear Size Bucket | Categorical | Small, Average, Large, Giant |
| 9 | | Nuclear Major Axis | Numeric | Maximum Diameter of Nucleus (DAPI Channel) |
| 10 | | Nuclear Minor Axis | Numeric | Minimum Diameter of Nucleus (DAPI Channel) |
| 11 | Nuclear Shape | Nuclear Circularity | Numeric | Nuclear Shape |
| 12 | | Nuclear Solidity | Numeric | Nuclear Shape |
| 13 | Nuclear Texture | Nuclear Entropy | Numeric | Nuclear Texture |
| 14 | | Nuclear Speckles | Numeric | # of DAPI Dots |
| 15 | Nuclear Artifacts | # Nucleoli | Numeric | # of Nucleoli |
| 16 | | nucleoli bin | Categorical | 0 Nucleoli, 1 Nucleoli, > 1 Nucleoli |
| 17 | Cytoplasmic Cell Size | max cell area | Numeric | Maximum Cell Area of CK, AR, DAPI Channels |
| 18 | | Cell Size | Categorical | Very Small, Small, Average, Large, Giant |
| 19 | | cyto. maj. axis (um) | Numeric | Maximum Diameter of Cytoplasm (CK or AR Channel) |
| 20 | | cyto. min. axis | Numeric | Minimum Diameter of Cytoplasm (CK or AR Channel) |
| 21 | Cytoplasmic Shape | Cytoplasmic Circularity | Numeric | Cytoplasmic Shape |
| 22 | | Cytoplasmic Solidity | Numeric | Cytoplasmic Shape |
| 23 | CK Texture | CK Speckles | Numeric | # of Cytokeratin Dots |
| 24 | | dotck bin | Categorical | Negative/Positive for Dot-Like CK Pattern |
| 25 | Area Ratio | Nuclear/Cytoplasmic Area Ratio | Numeric | Area Ratio |

As disclosed herein, NEPC patient CTCs demonstrated significant differences in CK, AR and morphological characteristics when compared to CRPC (Figures 4-8). Specifically, the frequency of CTCs not expressing epithelial markers (CK- and dim CK), with smaller less circular cells, larger nuclear/cytoplasmic ratios and a higher frequency of more prominent macronucleoli were greater in NEPC CTCs. Differences in these characteristics indicate NEPC patients having a higher frequency of CTCs with characteristics consistent with mesenchymal stem cell like characteristics resulting from EMT.

Disclosed is also a method for detecting transformation of adenocarcinoma into NEPC in a subject afflicted with prostate cancer comprising (a) performing a direct analysis comprising immunofluorescent staining and morphological characterization of nucleated cells in a blood sample obtained from the patient to detect circulating tumor cells (CTC); (b) determining presence or absence of a CTC subpopulation associated with NEPC comprising detecting a measurable feature of a panel of morphological and protein biomarkers, wherein the presence of the CTC subpopulation associated with NEPC is indicative of NEPC, and (c) repeating steps (a) and (b), wherein emergence of the presence of the CTC population associated with NEPC indicates transformation of adenocarcinoma into NEPC. In some cases, the subject is undergoing hormone treatment. The emergence of the CTC population associated with NEPC may predict resistance to hormone treatment, and inform a subsequent decision to discontinue hormone treatment and/or to initiate cytotoxic therapy. The emerging CTC population associated with NEPC that indicates the transformation of adenocarcinoma into NEPC comprises high CTC heterogeneity and a high prevalence of NEPC specific CTCs.

Before NEPC develops, metastatic tumors often show mixed features with both adenocarcinoma and neuroendocrine carcinoma cells present. There are no reliable serum markers to identify patients transforming to NEPC and the incidence of CTCs in these patients is unknown. Detection of NEPC has clinical implications, as NEPC patients would not be expected to respond well to currently approved potent AR targeted therapies for CRPC and may be selected for NEPC directed therapies.

Described is further a method for detecting atypical CRPC in a patient afflicted with prostate cancer comprising (a) performing a direct analysis comprising immunofluorescent staining and morphological characterization of nucleated cells in a blood sample obtained from the patient to detect circulating tumor cells (CTC), and (b) determining presence or absence of a CTC subpopulation associated with atypical CRPC comprising detecting a measurable feature of a panel of morphological and protein biomarkers, wherein the presence of the CTC subpopulation associated with atypical CRPC is indicative of atypical CRPC. In some cases, biomarkers observed in the CTC subpopulation associated with atypical CRPC comprise high CTC heterogeneity and a high prevalence of NEPC specific CTCs. Atypical CRPC exhibits features suggesting NEPC transition, including PSA<1ng/ml, elevated serum chromogranin, and/or visceral progression in absence of PSA progression.

As described further below, CTCs, which are molecularly similar to metastatic biopsies, can be detected by high definition imaging of plated nucleated cells in blood samples from prostate cancer patients. Methods for detection and characterization of these CTCs based on a direct analysis comprising immunofluorescent staining and morphological characteristics of nucleated cells can be useful for earlier detection of NEPC than presently available methods and can further inform the course of treatment of the patient based on phenotypic and/or molecular characterization of the CTCs.

The diagnosis of NEPC can be complex as there is a spectrum of morphologies seen in advanced prostate cancer with AR positive adenocarcinoma and pure AR negative small cell carcinoma representing the extreme phenotypes. Often times, metastatic biopsies reveal mixed features with both adenocarcinoma and neuroendocrine carcinoma observed and/or variable AR or neuroendocrine marker protein expression. The clinical significance of mixed tumors is less clear and treatment decisions are often individualized based on a combination of pathologic and clinical features. Furthermore, for patients with atypical clinical presentation such as rapid radiographic progression in setting of a low or modestly elevated PSA or elevated serum chromogranin, platinum-based therapies are sometimes considered even in the absence of neuroendocrine morphology on biopsy. Another challenge in the diagnosis of NEPC is that metastatic biopsies are not always feasible for patients suffering from advanced prostate cancer, may carry additional risks for the patient including complications from biopsy procedure or delay of initiation of appropriate systemic therapy for NEPC. A noninvasive marker to detect NEPC progression and simultaneously capture intratumoral heterogeneity is an unmet clinical need.

It must be noted that, as used in this specification and the appended claims, the singular forms "a", "an" and "the" include plural referents unless the content clearly dictates otherwise. Thus, for example, reference to "a CTC" includes a mixture of two or more CTCs, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

The term "patient," as used herein preferably refers to a human, but also encompasses other mammals. It is noted that, as used herein, the terms "organism," "individual," "subject," or "patient" are used as synonyms and interchangeably.

As used herein, the term "circulating tumor cell" or "CTC" is meant to encompass any rare cell that is present in a biological sample and that is related to prostate cancer. CTCs, which can be present as single cells or in clusters of CTCs, are often epithelial cells shed from solid tumors found in very low concentrations in the circulation of patients. CTCs include "traditional CTCs," which are cytokeratin positive (CK+), CD45 negative (CD-), contain a DAPI nucleus, and are morphologically distinct from surrounding white blood cells. The term also encompasses "non-traditional CTCs" which differ from a traditional CTC in at least one characteristic. Non-traditional CTCs include the five CTC subtypes, including CTC clusters, CK negative (CK⁻) CTCs that are positive at least one additional biomarker that allows classification as a CTC , small CTCs, nucleoli ⁺ CTCs and CK speckled CTCs. As used herein, the term "CTC cluster" means two or more CTCs with touching cell membranes.

In its broadest sense, a biological sample can be any sample that contains CTCs. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains nucleated cells and encompasses any material in which CTCs can be detected. A sample can be, for example, whole blood, plasma, saliva or other bodily fluid or tissue that contains cells.

In the invention, the biological sample is a blood sample. As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulation that are distinguishable by methods well known in the art (*e.g.,* FACS, immunohistochemistry). For example, a blood sample can contain populations of non-nucleated cells, such as erythrocytes (*e.g.*, 4-5 million/µl) or platelets (150,000-400,000 cells/µl), and populations of nucleated cells such as WBCs (*e.g.,* 4,500 - 10,000 cells/µl), CECs or CTCs (circulating tumor cells; *e.g.,* 2-800 cells/). WBCs may contain cellular subpopulations of, *e.g*., neutrophils (2,500-8,000 cells/µl), lymphocytes (1,000-4,000 cells/µl), monocytes (100-700 cells/µl), eosinophils (50-500 cells/µl), basophils (25 - 100 cells/ µl) and the like. The samples of this disclosure are non-enriched samples, *i.e.,* they are not enriched for any specific population or subpopulation of nucleated cells. For example, non-enriched blood samples are not enriched for CTCs, WBC, B-cells, T-cells, NK-cells, monocytes, or the like.

In some embodiments, the sample is a blood sample, obtained from a subject who has been diagnosed with prostate cancer, including without limitation, mCRPC, based on tissue or liquid biopsy and/or surgery or clinical grounds. In some embodiments, the blood sample is obtained from a subject showing a clinical manifestation of prostate cancer, including without limitation, mCRPC, well known in the art or who presents with any of the known risk factors for prostate cancer. In other embodiments, the biological sample is obtained from a healthy subject or a subject deemed to be at high risk for prostate cancer and/or metastasis of existing prostate cancer based on art known clinically established criteria including, for example, age, race, family and history.

As used herein, the term "direct analysis" means that the CTCs are detected in the context of all surrounding nucleated cells present in the sample as opposed to after enrichment of the sample for CTCs prior to detection. In some embodiments, the methods comprise microscopy providing a field of view that includes both CTCs and at least 200 surrounding white blood cells (WBCs).

A fundamental aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables characterization of CTCs, including subtypes of CTCs described herein, that cannot be achieved with other, enrichment-dependent CTC detection methods and that enables the identification and analysis of morphological and protein biomarkers indicative of the presence of a CTC subpopulation associated with NEPC in the context of the claimed methods.

The methods disclosed and provided herein enable detection of NEPC in a patient afflicted with prostate cancer and make it possible to distinguish between different patient groups in the resistance setting in order to tailor subsequent treatment more precisely and effectively. The methods of the invention further allow for resistance monitoring of a prostate cancer patients by enabling detection of an emergence of NEPC in a patient afflicted with prostate cancer. The rapid evolution of drug therapies in prostate cancer has vastly improved upon the use of docetaxel since its pivotal US Food and Drug Administration (FDA) approval in 2004 and has brought about a new era where progress has been made beyond the use of androgen deprivation therapy (ADT) with the addition of novel hormonal agents, immunotherapy, second-line chemotherapy as well as radiopharmaceuticals. The choice of sequencing currently relies on patient profiles, whether symptoms of metastatic disease exist or not. While survival outcomes are undeniably improved with the use of these therapies, disease will ultimately progress on each regimen.

Androgens in the form of testosterone or the more potent dihydrotestosterone (DHT) have been well-defined drivers of progression of prostate cancer and differentiation of the prostate gland. As such, the backbone of treatment for advanced prostate cancers was established decades ago when castration in the form of surgical orchiectomy achieved significant prostate tumor regression. Since then, substitution to chemical castration has been employed mostly due to patient preference. ADT has therefore become the standard systemic treatment for locally advanced or metastatic prostate cancer. While ADT is almost always effective in most patients, disease progression to castration resistance inevitably occurs. It is now increasingly recognized that the androgen receptor (AR) remains overexpressed despite seemingly castrate levels of testosterone, since alternative receptors may activate the AR or other target genes may help perpetuate the castrate-resistant phenotype, hence the term "castration-resistance" has become widely adopted in the literature. The enhanced understanding of the role of these androgens in stimulating the growth of prostate cancer has led to the development and approval of a newer generation anti-androgen hormonal therapy drugs such as Zytiga (arbiterone), which blocks androgen production, and Xtandi (enzalutamide), an androgen receptor (AR) inhibitor. However, in a small percentage of patients, the treatment with hormone therapy converts or transforms adenocarcinoma into NEPC, which is characterized by its lack of dependence upon either androgen or androgen receptor for its growth. Consequently, continued hormone therapy is unlikely to benefit these men. As described herein, the methods of the invention enable detection of NEPC in a patient afflicted with prostate cancer and make it possible to distinguish between different patient groups in the resistance setting in order to tailor subsequent treatment more precisely and effectively. The methods of the invention further allow for resistance monitoring of a prostate cancer patients by enabling detection of an emergence of NEPC in a patient afflicted with prostate cancer. Current treatment for confirmed or suspected NEPC is typically cytotoxic chemotherapy, often using a platinum-based regimen similar to those used to treat other neuroendocrine small cell carcinomas. The most commonly used chemotherapy drug to treat prostate cancer is docetaxel (Taxotere®). Other drugs that can be used include, without limitation, mitoxantronepaclitaxel (Taxol®) and cabazitaxel.

In some aspects, the disclosure relates to a method for detecting transformation of adenocarcinoma into NEPC in a subject afflicted with prostate cancer comprising (a) performing a direct analysis comprising immunofluorescent staining and morphological characterization of nucleated cells in a blood sample obtained from the patient to detect circulating tumor cells (CTC); (b) determining presence or absence of a CTC subpopulation associated with NEPC comprising detecting a measurable feature of a panel of morphological and protein biomarkers, wherein the presence of the CTC subpopulation associated with NEPC is indicative of NEPC, and (c) repeating steps (a) and (b), wherein emergence of the presence of the CTC population associated with NEPC indicates transformation of adenocarcinoma into NEPC. In some cases, the subject is undergoing hormone treatment.

In other aspects, the disclosure relates to a method for monitoring disease progression in a subject afflicted with prostate cancer comprising (a) performing a direct analysis comprising immunofluorescent staining and morphological characterization of nucleated cells in a blood sample obtained from the patient to detect circulating tumor cells (CTC); (b) determining presence or absence of a CTC subpopulation associated with NEPC comprising detecting a measurable feature of a panel of morphological and protein biomarkers, wherein the presence of the CTC subpopulation associated with NEPC is indicative of NEPC, and (c) repeating steps (a) and (b), wherein emergence of the presence of the CTC population associated with NEPC indicates progression of the prostate cancer to NEPC.

In related aspects, the disclosure relates to a method for resistance monitoring in a subject afflicted with prostate cancer comprising (a) performing a direct analysis comprising immunofluorescent staining and morphological characterization of nucleated cells in a blood sample obtained from the patient to detect circulating tumor cells (CTC); (b) determining presence or absence of a CTC subpopulation associated with NEPC comprising detecting a measurable feature of a panel of morphological and protein biomarkers, wherein the presence of the CTC subpopulation associated with NEPC is indicative of NEPC, and (c) repeating steps (a) and (b), wherein emergence of the presence of the CTC population associated with NEPC indicates resistance to hormone treatment.

In some cases, the patient is undergoing hormone treatment. The hormone treatment may be anti-androgen hormonal therapy, for example, Zytiga (arbiterone), which blocks androgen production, and Xtandi (enzalutamide), an androgen receptor (AR) inhibitor. In additional embodiments, the emergence of the CTC population associated with NEPC predicts resistance to hormone treatment and informs a subsequent decision to discontinue hormone treatment and/or to initiate cytotoxic therapy. In some cases, the subsequent treatment decision upon detecting NEPC is cytotoxic chemotherapy with a platinum-based regimen, for example and without limitation, docetaxel (Taxotere®), mitoxantronepaclitaxel (Taxol®) and cabazitaxel.

In some embodiments, the method for detecting NEPC in a patient afflicted with prostate cancer can further encompass individual patient risk factors, clinical, biopsy or imaging data, which includes any form of imaging modality known and used in the art, for example and without limitation, by X-ray computed tomography (CT), ultrasound, positron emission tomography (PET), electrical impedance tomography and magnetic resonance (MRI). It is understood that one skilled in the art can select an imaging modality based on a variety of art known criteria. Additionally, the method of the invention, can optionally encompass one or more one or more individual risk factors that can be selected from the group consisting of, for example, age, race, family history, clinical history and/or data.

Risk factors for NEPC in the context of clinical data further include, for example, rapidly progressive disease or visceral disease such as liver metastases or brain metastases in the setting of low prostate-specific antigen (PSA). In those cases, biopsies can be performed to confirm or rule out NEPC and methods for detecting NEPC in a patient afflicted with prostate cancer can further take encompass as a risk factor the resultant biopsy data. It is understood that one skilled in the art can select additional individual risk factors based on a variety of art known criteria. As described herein, the methods of the invention can encompass one or more individual risk factors. Accordingly, biomarkers can include, without limitation, imaging data, clinical data, biopsy data, and individual risk factors. As described herein, biomarkers also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.*, glycoproteins, ribonucleoproteins, lipoproteins) as well as portions or fragments of a biological molecule.

Direct analysis of CTCs according to the methods of the invention includes both morphological features and immunofluorescent features. As will be understood by those skilled in the art, biomarkers can include a biological molecule, or a fragment of a biological molecule, the change and/or the detection of which can be correlated, individually or combined with other measurable features, with NEPC. CTCs, which can be present a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1:1,000 in a blood cell population, *e.g.*, an abundance of less than 1:5,000, 1:10,000, 1:30,000, 1:50:000, 1:100,000, 1:300,000, 1:500,000, or 1:1,000,000. In some embodiments, the a CTC has an abundance of 1:50:000 to 1:100,000 in the cell population.

The samples of this disclosure may be obtained by any means, including, *e.g*., by means of solid tissue biopsy or fluid biopsy (*see, e.g.,* Marrinucci D. *et al.,* 2012, Phys. Biol. 9 016003). Briefly, in particular embodiments, the process can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (*e.g.*, procedures for drawing and processing whole blood). In some embodiments, a blood sample is drawn into anti-coagulent blood collection tubes (BCT), which may contain EDTA or Streck Cell-Free DNA™. In other embodiments, a blood sample is drawn into CellSave® tubes (Veridex). A blood sample may further be stored for up to 12 hours, 24 hours, 36 hours, 48 hours, or 60 hours before further processing.

The methods of this disclosure may comprise an intitial step of obtaining a white blood cell (WBC) count for the blood sample. The WBC count may be obtained by using a HemoCue® WBC device (Hemocue, Ängelholm, Sweden). The WBC count may be used to determine the amount of blood required to plate a consistent loading volume of nucleated cells per slide and to calculate back the equivalent of CTCs per blood volume.

The methods of this disclosure may further comprise an initial step of lysing erythrocytes in the blood sample. The erythrocytes may be lysed, *e.g.,* by adding an ammonium chloride solution to the blood sample. A blood sample may be subjected to centrifugation following erythrocyte lysis and nucleated cells are resuspended, *e.g*., in a PBS solution.

In some embodiments, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 µm. In some embodiments, the material is a film. In some embodiments the material is a glass slide. In certain embodiments, the method encompasses an initial step of depositing nucleated cells from the blood sample as a monolayer on a glass slide. The glass slide can be coated to allow maximal retention of live cells (*See, e.g.,* Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto the glass slide. In some embodiments, the glass slide and immobilized cellular samples are available for further processing or experimentation after the methods of this disclosure have been completed.

In some embodiments, the methods disclosed and provided herein comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI). In some embodiments, immunofluorescent staining of nucleated cells comprises pan cytokeratin (CK), cluster of differentiation (CD) 45 and DAPI. In some embodiments further described herein, CTCs comprise distinct immunofluorescent staining from surrounding nucleated cells. In some embodiments, the distinct immunofluorescent staining of CTCs comprises DAPI (+), CK (+) and CD 45 (-). In some embodiments, the identification of CTCs further comprises comparing the intensity of pan cytokeratin fluorescent staining to surrounding nucleated cells. In some embodiments, the CTCs are CK- CTCs, that are identified as CTC based on other characteristics. As described herein, CTCs detected encompass traditional CTCs. cytokeratin negative (CK⁻) CTCs, small CTCs, and CTC clusters. In some embodiments, the CTC detection and analysis is accomplished by fluorescent scanning microscopy to detect immunofluorescent staining of nucleated cells in a blood sample. Marrinucci D. et al., 2012, Phys. Biol. 9 016003).

In particular embodiments, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified, for example, as DAPI (+), CK (+) and CD 45 (-). In the methods described herein, the CTCs comprise distinct immunofluorescent staining from surrounding nucleated cells.

As described herein, CTCs encompass traditional CTCs, also referred to as high definition CTCs (HD-CTCs). Traditional CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during CTC enumeration as previously described. Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous. In some embodiments, the morphological characteristics of a CTC detected in the methods of the invention comprise one or more of the group consisting of nucleus size, nucleus shape, presence of holes in nucleus, cell size, cell shape and nuclear to cytoplasmic ratio, nuclear detail, nuclear contour, presence or absence of nucteoli, quality of cytoplasm and quantity of cytoplasm.

As described herein, the methods for detecting NEPC in a patient afflicted with prostate cancer encompass determining the presence or absence of a CTC subpopulation associated with NEPC comprising detecting a measurable feature of a panel of morphological and protein biomarkers, wherein the presence of the CTC subpopulation associated with NEPC is indicative of NEPC. In particular embodiments, the protein biomarkers in step (b) comprise the Androgen Receptor (AR) and pan cytokeratin (CK), and the morphological biomarkers unique to the CTC subpopulation associated with NEPC comprise small size and the presence of nucleoli (nucleoli⁺). In additional embodiments, determining the presence of a CTC subpopulation associated with NEPC comprises analysis of the CTCs at the single cell level.

While traditional CTCs can be immunofluorescently identified as comprising DAPI (+), CK (+) and CD 45 (-) cells, the methods described can be practiced with any other biomarkers that one of skill in the art selects for detecting traditional and non-traditional CTCs in a biological sample. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide.

A person skilled in the art will appreciate that a number of methods can be used to detect and analyze CTCs, including microscopy based approaches, including fluorescence scanning microscopy (*see, e.g.,* Marrinucci D. et al., 2012, Phys. Biol. 9 016003), mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunopercipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will further appreciate that the presence or absence of protein biomarkers may be detected using any class of marker-specific binding reagents known in the art, including, *e.g*., antibodies, aptamers, fusion proteins, such as fusion proteins including protein receptor or protein ligand components, or biomarker-specific small molecule binders. In some embodiments, the presence or absence of AR, CK or CD45 is determined by an antibody.

The antibodies of this disclosure bind specifically to a protein biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some embodiments, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers when practicing the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.,* fluorescein, fluorescein isothiocyanate (FITC), Oregon Green™, rhodamine, Texas red, tetrarhodimine isothiocynate (TRITC), Cy3, Cy5, Alexa Fluor® 647, Alexa Fluor® 555, Alexa Fluor® 488), fluorescent markers (*e.g*., green fluorescent protein (GFP), phycoerythrin, etc.), enzymes (*e.g*., luciferase, horseradish peroxidase, alkaline phosphatase, etc.), nanoparticles, biotin, digoxigenin, metals, and the like.

For mass-sectrometry based analysis, differential tagging with isotopic reagents, *e.g*., isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I, or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for epithelial cells In some embodiments, the immunofluorescent makers comprise a marker specific for white blood cells (WBCs). In some embodiments, one or more of the immunofluorescent markers comprise CD45 and CK.

In some embodiments, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, CTCs comprise distinct morphological characteristics compared to surrounding nucleated cells. In some embodiments, the morphological characteristics comprise nucleus size, nucleus shape, cell size, cell shape, and/or nuclear to cytoplasmic ratio. In some embodiments, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC. In particular embodiments, the morphological characteristics analyzed in the methods of the invention comprise one or more of the group consisting of nucleus size, nucleus shape, presence of holes in nucleus, cell size, cell shape and nuclear to cytoplasmic ratio, nuclear detail, nuclear contour, presence or absence of nucleoli, quality of cytoplasm and quantity of cytoplasm.

Detection and analysis of CTCs can be performed with any suitable microscopic method known in the art. In some embodiments, the method is performed by fluorescent scanning microscopy. In certain embodiments the microscopic method provides high-resolution images of CTCs and their surrounding WBCs (*see, e.g.,* Marrinucci D. et al., 2012, Phys. Biol. 9 016003)). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

In some embodiments, the method of the invention includes detecting biomarkers AR, CK and CD 45. A biomarker is considered present in a cell if it is detectable above the background noise of the respective detection method used (*e.g.,* 2-fold, 3-fold, 5-fold, or 10-fold higher than the background; *e.g*., 2σ or 3σ over background). In some embodiments, a biomarker is considered absent if it is not detectable above the background noise of the detection method used (*e.g.,* <1.5-fold or <2.0-fold higher than the background signal; *e.g.,* <1.5σ or <2.0σ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (*i.e.,* the marker is considered present) if its fluorescent signal for the respective marker is significantly higher than the fixed background signal (*e.g.,* 2-fold, 3-fold, 5-fold, or 10-fold higher than the background; *e.g.,* 2σ or 3σ over background). For example, a nucleated cell is considered CD 45 positive (CD 45⁺) if its fluorescent signal for CD 45 is significantly higher than the background signal. A cell is considered negative for an immunofluorescent marker (*i.e.,* the marker is considered absent) if the cell's fluorescence signal for the respective marker is not significantly above the background signal (*e.g.,* <1.5-fold or <2.0-fold higher than the background signal; *e.g.,* <1.5σ or <2.0σ over background).

Typically, each microscopic field contains both CTCs and WBCs. In certain embodiments, the microscopic field shows at least 1, 5, 10, 20, 50, or 100 CTCs. In certain embodiments, the microscopic field shows at least 10, 25, 50, 100, 250, 500, or 1,000 fold more WBCs than CTCs. In certain embodiments, the microscopic field comprises one or more CTCs or CTC clusters surrounded by at least 10, 50, 100, 150, 200, 250, 500, 1,000 or more WBCs.

In some embodiments of the methods described and provided herein, detection of CTCs comprises enumeration of CTCs that are present in the blood sample. In some embodiments, the methods described herein encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some embodiments, the CTCs detected in a biological sample comprise distinct subtypes of CTCs, including non-traditional CTCs. In some embodiments, the methods described herein encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular embodiment, the methods described herein encompass detection of at least 1 CTC cluster/mL of blood

In some embodiments, the method for detecting neuroendocrine prostate cancer (NEPC) in a patient afflicted with prostate cancer comprising molecular analysis of the CTCs further comprise molecular characterization of the CTCs, for example, by fluorescence *in situ* hybridization (FISH). In additional embodiments, the FISH analysis detects amplification of aurora kinase A (*AURKA*) gene. In further embodiments, the FISH analysis detects amplification of MYCN (*N-MYC*) gene.

Standard molecular biology techniques known in the art and not specifically described are generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Md. (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998). Polymerase chain reaction (PCR) can be carried out generally as in PCR Protocols: A Guide to Methods and Applications, Academic Press, San Diego, Calif. (1990). Any method capable of determining a DNA copy number profile of a particular sample can be used for molecular profiling provided the resolution is sufficient to identify the biomarkers. The skilled artisan is aware of and capable of using a number of different platforms for assessing whole genome copy number changes at a resolution sufficient to identify the copy number of the one or more biomarkers.

In situ hybridization assays are well known and are generally described in Angerer et al., Methods Enzymol. 152:649-660 (1987). In an in situ hybridization assay, cells, e.g., from a biopsy, are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters. FISH (fluorescence in situ hybridization) uses fluorescent probes that bind to only those parts of a sequence with which they show a high degree of sequence similarity.

FISH is a cytogenetic technique used to detect and localize specific polynucleotide sequences in cells. For example, FISH can be used to detect DNA sequences on chromosomes. FISH can also be used to detect and localize specific RNAs, e.g., mRNAs, within tissue samples. In FISH uses fluorescent probes that bind to specific nucleotide sequences to which they show a high degree of sequence similarity. Fluorescence microscopy can be used to find out whether and where the fluorescent probes are bound. In addition to detecting specific nucleotide sequences, e.g., translocations, fusion, breaks, duplications and other chromosomal abnormalities, FISH can help define the spatial-temporal patterns of specific gene copy number and/or gene expression within cells and tissues.

In some embodiments, the method of the invention for detecting NEPC in a patient afflicted with prostate cancer encompasses the use of a predictive model. In further embodiments, the method encompasses comparing s a measurable feature with a reference feature. As those skilled in the art can appreciate, such comparison can be a direct comparison to the reference feature or an indirect comparison where the reference feature has been incorporated into the predictive model. In further embodiments, analyzing a measurable feature in a method for detecting NEPC in a patient afflicted with prostate cancer encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression. In additional embodiments, the detection of NEPC in a patient afflicted with prostate cancer is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, *e.g.* AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, *e.g.* log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

In some embodiments, the method of the invention for detecting NEPC in a patient afflicted with prostate cancer has a specificity of >60%, >70%, >80%, >90% or higher. In additional embodiments, the method disclosed herein for detecting NEPC in a patient afflicted with prostate cancer has a specificity >90% at a classification threshold of 7.5 CTCs/mL of blood.

The following examples are provided by way of illustration, not limitation.

### EXAMPLES

### Example 1. Identification and molecular characterization of the CTCs in NEPC.

This experiment demonstrates detection of CTCs from patients with NEPC and similarity of these CTCs with metastatic biopsies.

Blood from 11 consecutive patients (pts) with metastatic prostate cancer and clinical or pathologic features suggestive of NEPC were collected and shipped to Epic Sciences, where cells were identified utilizing Epic CTC collection and detection process (Figure 1). Traditional CTCs were identified as CK+CD45- cells with intact DAPI nuclei and after pathologist review of their morphology. Candidate CK- CTC populations were identified as CK-CD45- that were morphologically malignant. Small nuclear size candidate CTCs were identified as CK+CD45- cells with diameters similar to or smaller than that of a typical white blood cells (WBCs). Candidate CTC were evaluated with prostate cancer relevant biomarkers, including androgen receptor (AR) expression by immunofluorescence (IF), a subset of CTCs were stained for EpCAM and Aurora kinase FISH. Exome and RNA-sequencing were performed in patient-matched metastatic biopsies obtained at same time of CTC analysis in 8 cases.

With regard to patient demographics, patients were classified as having pathologically confirmed NEPC arising de novo (n=2) or after treatment (n=4), or clinically diagnosed NEPC based on CRPC with highly aggressive visceral progression and/or PSA <1 ng/ml (n=5). 5 NEPC patients were previously treated with abiraterone and/or enzalutamide. Age range was 62-86 yrs. Sites of metastases included liver (7/11), bone (10/11), lymph nodes (8/11), pleura (1/11), lungs (2/11). Serum PSA, CellSearchⓇ CTCs, and serum NE markers were variable including 5/11 patients with PSA<1 ng/ml (range 0.02-9.39) and 4/6 evaluated pts with CTC count of 0-3 (range 0-94). Metastatic tumor biopsies were evaluated in all cases including one rapid autopsy with histology ranging from poorly differentiated carcinoma to small cell carcinoma. Neuroendoocrine markers (NSE, chromogranin, synaptophysin) showed strong expression by immunohistochemistry in 6/11 cases. 6/8 evaluated tumors displayed amplification of Aurora kinase A by FISH. Patients were subsequently treated with platinum (4/11), AURKA inhibitor (4/11), hormonal therapy (2/11), or progressed to death within 1 day of CTC collection (rapid autopsy case).

### Example 2. Further Studies on Identification and molecular characterization of the CTCs in NEPC

Neuroendocrine prostate cancer (NEPC) is an aggressive androgen independent variant of prostate cancer that most commonly arises in later stages of castration resistant prostate cancer (CRPC) as a mechanism of treatment resistance. Androgen receptor (AR) expression is typically low or absent in NEPC and the genes Aurora kinase A (AURKA) and N-Myc (MYCN) are frequently amplified (Beltran et al, Cancer Discov. 1(6):487-95 (2011); Mosquera et al., Neoplasia. 15(1):1-10. (2013)). There are no reliable serum markers to identify patients that are transforming to NEPC and incidence of CTCs in these patients is unknown. Detection of NEPC has clinical implications as these patients would not be expected to respond well to currently approved potent AR targeted therapies for CRPC. Circulating tumor cells (CTCs) are traditionally defined as EpCAM/cytokeratin positive (CK+) cells, CD45-, and morphologically distinct. However, recent evidence suggests that other populations of CTC candidates exist, including cells that are EpCAM/cytokeratin negative (CK-) or cells smaller in size than traditional CTCs. CTC positive selection techniques that isolate CTCs based on size, density, or EpCAM positivity may miss CTC subpopulations. These studies were aimed to molecularly characterize CTCs from patients with NEPC utilizing the Epic Sciences platform, which performs no physical selection, and to correlate CTC results with patient- matched clinical, molecular, and pathologic features.

Blood from 11 consecutive patients (pts) with metastatic prostate cancer and clinical or pathologic features suggestive of neuroendocrine prostate cancer (NEPC) were collected and shipped to Epic Sciences, where cells were identified utilizing Epic CTC collection and detection process (see Figure 1). Traditional CTCs were identified as CK+CD45- cells with intact DAPI nuclei and after pathologist review of their morphology. Candidate CK- CTC populations were identified as CK-CD45- that were morphologically malignant. Small nuclear size candidate CTCs were identified as CK+CD45- cells with diameters similar to or smaller than that of a typical white blood cells (WBCs). Candidate CTC were evaluated with prostate cancer relevant biomarkers, including androgen receptor (AR) expression by immunofluorescence (IF), a subset of CTCs were stained for EpCAM and Aurora kinase FISH. Exome and RNA-sequencing were performed in patient-matched metastatic biopsies obtained at same time of CTC analysis in 8 cases.

Novel CTC subpopulations in NEPC were identified. As shown in Figure 2A, low CK CTCs with cancer related morphology are seen which have no AR and no EpCAM expression. CTCs from NEPC patients also exhibit unique spindle-like morphology. As shown in Figure 2B, AuroraK amplification was seen in tissue and CTC samples. Samples from Patient #6 were evaluated. Figure 2B shows castration resistant tumor tissue, where hematoxylin-eosin (H&E) shows poorly differentiated carcinoma, and Aurora kinase A and N-myc amplified by FISH. Figure 2C shows patient matched circulating tumor cells (CK+, CD45-, AR-), and Aurora kinase A also amplified by FISH (Aurora kinase DNA probes; Empire Genomics, Buffalo NY).

The sensitivity of Epic CTC platform versus CellSearch® system (Janssen Diagnostics; Raritan, NJ). Matched blood samples were processed utilizing CellSearch® and Epic Sciences CTC platform (see Figure 3A). Figure 3A shows Epic Sciences CTCs extrapolated to 7.5 ml versus CellSearch® CTCs per 7.5 mL. Epic CTCs were measured from 1 mL and extrapolated to 7.5 ml of blood. AR expression and distribution of CTCs was determined. Figure 3B shows AR expression per CTC or CTC cluster plotted for each patient. It was observed that traditional CTCs harbor low or no AR expression. CK expression and distribution of CTCs was also determined. Figure 3C shows CK expression per CTC or CTC cluster plotted for each patient. It was observed that CK expression on NEPC CTCs was significantly lower than CK expression seen in control cell lines.

Identification of patients with CRPC progressing towards an AR independent NEPC phenotype remains challenging, especially in the absence of metastatic tumor biopsy. Serum PSA, serum NE markers and CTC count by CellSearch® are unreliable. Epic CTCs from patients with NEPC are smaller in size and show unique spindle like morphology, low cytokeratin expression, and lack AR and EpCAM expression. Epithelial plasticity potentially arising from EMT may explain the lack of detection using conventional CTC assays. These results show that the Epic Sciences CTC platform is capable of detecting CTCs from patients with NEPC and CTCs are molecularly similar to metastatic biopsies. Therefore, Epic CTCs are useful in the earlier detection of NEPC and can inform patient selection for therapy.

### Example 3. CTCs from NEPC have unique phenotypes compared with CTCs from mCRPC

This example demonstrates that CTCs from NEPC have unique phenotypes compared with CTCs from mCRPC.

Sixteen (16) patients with tissue confirmed NEPC and 8 pts with mCRPC had blood collection for CTC analysis utilizing the Epic Sciences platform. Epic analysis included identification of traditional CTCs (CK+, CD45- cells, with intact nuclei, morphology distinct), CK- CTCs (CK-, CD45-, intact nuclei, morphology distinct), small CTCs (CK+, CD45-, intact nuclei, small cell size), and CTC clusters. The CTCs were examined at the single cell level for CTC size, shape, CK and AR expression. Advanced digital pathology algorithms measured size and shape measurements of CTCs.

CTCs from NEPC patients demonstrated strong statistical differentiation from mCRPC patients, with unique CTC morphology and protein chemistry that was not seen in mCRPC CTCs. NEPC CTCs had increase prevalence of unique CTC phenotypes including: small size, AR negativity, and presence of nucleoli (nucleoli+). Unique cell types enabled a multivariate biomarker that is strongly associated with CTCs exclusive to tissue confirmed NEPC.

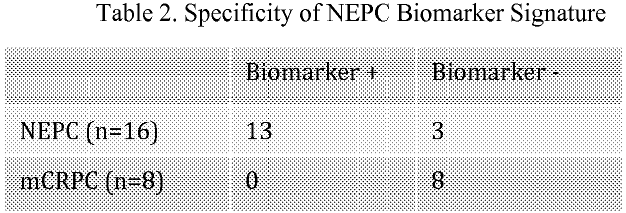

CTCs from NEPC have unique phenotypes compared with those from mCRPC. If confirmed, the utilization of a liquid biopsy to diagnose NEPC may enable earlier identification of patients prone to visceral metastasis, and intervention to cytotoxic therapeutics. Additionally, identification of patients with NEPC could help for patient selection for AR vs. NEPC targeted therapeutics.

### Example 4. Detection and Characterization of CTCs from patients with NEPC

This example demonstrates detection of CTCs from patients with NEPC. This Example further demonstrates that that CTCs from NEPC patients have unique multivariate morphologic and protein signatures from CRPC CTCs enabling the NEPC diagnostic signature.

CTC collection. Under an IRB approved protocol at WCMC, patients with metastatic NEPC and CRPC were prospectively enrolled and blood was collected for CTC analysis. NEPC was defined histologically by the presence of predominantly small cell carcinoma on tumor biopsy; therefore, all NEPC patients had matched metastatic biopsy to confirm the diagnosis. CRPC was defined clinically as PSA or radiographic progression despite castrate levels of serum testosterone, with or without metastatic biopsy confirming prostate adenocarcinoma. CRPC patients were sub-classified as atypical CRPC if metastatic tumor biopsy showed adenocarcinoma but AR independent transition was suspected based on radiographic progression in the setting of a low PSA <1ng/ml, visceral progression in the absence of PSA progression (defined by Prostate Cancer Working Group 2 criteria) or elevated serum chromogranin A >3 fold higher than the upper limit of normal.

Clinical demographics including age, sites of metastases, prior therapies, serum PSA, serum neuroendocrine marker levels (chromogranin, NSE), and CellSearch® CTC count were collected. Blood (10 mL) from each subject was shipped to Epic Sciences within 48 hours and processed immediately on arrival. Red blood cells were lysed, approximately 3 million nucleated blood cells dispensed onto 10-16 glass microscope slides according to methods previously described (Hsieh et al. (2006) Biosens Bioelectron 21: 1893-1899; Marrinucci et al. (2007) Hum Pathol 38: 514-519; Marrinucci et al. (2012) Phys Biol 9: 016003) and placed at-80°C for long term storage. CTC slides stored at -80C using this approach are stable over 1 year.

CTC identification. CTCs were identified utilizing the Epic CTC collection and detection process (Figure 1) in batch. Two slides from each patient sample were thawed and subjected to an IF staining protocol to distinguish CTCs from WBCs as described previously (Marrinucci et al. (2012) Phys Biol 9: 016003; Marrinucci (2010) J Oncol 2010: 861341). The Epic assay utilizes antibodies targeting cytokeratins (CK), CD45, and the androgen receptor (AR). A 4',6-diamidino-2-phenylindole (DAPI) counterstain was also applied to identify cell nuclei. Stained slides were then imaged via a high speed imaging platform that images all 3M cells on each slide in less than 15 minutes. Captured images were analyzed by a proprietary software that characterizes each cell by over 90 parameters including cell size, cell shape, nuclear area, the presence of nucleoli, CK and AR expression, uniformity and cellular localization. CTC candidates are identified in an interactive report and subsequently reviewed by trained technicians. CK+/CD45- cells with intact, DAPI+ nuclei exhibiting tumor-associated morphologies were classified as traditional CTCs. CTCs with non-traditional characteristics were also analyzed, such as CK-/CD45- cells with morphological distinction and/or AR positivity, CK+/CD45- small cells, CTC clusters, CTCs with multiple nucleoli and apoptotic CTCs (identified by nuclear or cytoplasmic fragmentation).

CTC FISH analysis. From a subset of patients with sufficient CTCs and Aurora kinase amplification in tumor biopsy, CTCs were then further tested for Aurora Kinase amplification by FISH. Coverslips were removed from slides selected for FISH analysis, mounting medium was rinsed, and cells were fixed and dehydrated with formaldehyde and ethanol, respectively. After complete dehydration, a 1 color probe solution (Empire Genomics) targeting Aurora Kinase DNA sequences was applied across the entire deposition area of each slide, coverslips were applied, sealed and hybridized for 18-24 hours at 37°C. Slides were washed in a series of saline sodium citrate (SSC)/detergent (Igepal) solutions with increasing stringency, counterstained with DAPI, and mounted with an anti-fade mounting medium. Because the exact coordinates of every CTC are recorded, each CTC was then relocated and scored. 20 WBCs on each slide were also scored as internal controls.

Pathologic evaluation. Patient-matched metastatic tumor biopsies were reviewed in all cases by two anatomic genitourinary pathologists. Tumor morphology and pathologic characteristics including immunohistochemical marker staining for neuroendocrine markers (chromogranin, NSE, or synaptophysin) were assessed. IHC was quantified on scale 0-3 and overexpression was defined as any staining intensity seen of target cells above background. Fluorescent in situ hybridization (FISH) was used to evaluate for copy number gain of the aurora kinase A gene (AURKA) (BAC probe RP11-158017). Copy number gain of AURKA was defined as the presence of 3 to 4 copies on average for gene-specific signals per nuclei compared to two reference signals. At least 100 nuclei were evaluated per core/tissue section.

Statistical Analysis. CTC morphological/molecular data and clinical information were compiled into patient subtype datasets (based on tumor diagnosis; NEPC, mCRPC, atypical mCRPC) using KNIME, where the following characteristics were analyzed with single cell resolution: cytokeratin expression, AR expression, presence of clusters and various nuclear and cytoplasmic morphological features. Kernel density estimates (KDE) of each CTC characteristic were performed to provide univariate distributions across each aggregate subtype (NEPC, mCRPC or atypical mCRPC) further identifying NEPC specific CTC characteristics. Patient samples were analyzed for the frequency of cell types at calculated cell counts per mL of blood, and univariate distributions of CTC biomarkers were compared at the patient level for each diagnostic category. Supervised learning was performed using the Random Forest classifier algorithm (R package 'randomForest') set with 1,001 decision trees and configured to provide a probability output (Breiman, Random Forests. Machine Learning 45: 5-32(2001).

Leave-One-Out Cross-Validation. To evaluate the robustness of the Random Forest classifier applied to CTC datasets, leave-one-out cross validation was performed on samples from patients diagnosed with NEPC and CRPC. CTCs from patients diagnosed with atypical CRPC were removed from analysis; while CTCs from patients diagnosed with NEPC were labeled NEPC+, and CTCs from patients diagnosed with CRPC were labeled NEPC-. Leave-one-out cross-validation was performed at the blood sample level, where the dataset was partitioned into training sets and test sets as shown by the schematic for a single iteration in Figures 12-14. For each blood tube in the dataset, labeled CTCs from every other sample were used to train the classifier, and CTCs from the blood tube being evaluated were held-out as the test set. Next, CTCs from the test set are analyzed by the trained classifier, where the output is an estimated probability of class membership to NEPC+ and NEPC- for each CTC belonging to the held-out patient sample. This cycle repeats iteratively for each blood (patient) sample in the dataset, and the classifier output is collected at the end of each iteration.

Atypical CRPC Analysis. In the Leave-One-Out Cross-Validation, patients with atypical CRPC were excluded from the analysis. Here, all CRPC and NEPC were used as a training set, and the atypical CRPC were analyzed as the test set. The output of the classification is an estimated probability of class membership to NEPC+ and NEPC- for each CTC belonging to the atypical CRPC patient. Kernel density estimate curves were used to plot the distribution of NEPC+ class membership values for individual CTCs for each patient.

**Results.** Patient Characteristics. Circulating tumor cells from 27 metastatic prostate cancer patients, including 12 NEPC and 15 CRPC (including 5 atypical CRPC) were compared. Pathologic and clinical features, including diagnosis, sites of metastasis, and biopsy immunuhistochemical profiles, from included patients are summarized in Figure 16. In addition, common prostate cancer serum marker expression (PSA, CgA, NSE), often associated with disease progression, was analyzed from patient blood. No correlation in the expression of any biopsy or serum markers across patients in NEPC, mCRPC and atypical mCRPC subgroups was observed, further highlighting the unreliability of these markers to identify NEPC mCRPC and the heterogeneity of NEPC disease progression.

CTC Characteristics. NEPC patient CTCs demonstrated significant differences in CK, AR and morphological characteristics when compared to CRPC (Figures 4-8). Specifically, the frequency of CTCs not expressing epithelial markers (CK- and dim CK), with smaller less circular cells, larger nuclear/cytoplasmic ratios and a higher frequency of more prominent macronucleoli were greater in NEPC CTCs. Differences in these characteristics indicate NEPC patients having a higher frequency of CTCs with characteristics consistent with mesenchymal stem cell like characteristics potentially resulting from EMT. As shown in Figure 3, cell classification models demonstrated good specificity and sensitivity for identification of cell with these characteristics across the aggregate NEPC cohort (AUC=0.77-0.83).

NEPC CTC Frequency. For the diagnosis of patients with NEPC, serum markers are often unreliable indicators. In this study, serum prostate specific antigen (PSA), CellSearch CTC count, and serum chromogranin and neuron specific endonuclease (NSE) levels were variable (Figure 2). As an alternative to the NEPC markers described, enumeration of CTCs using both the CellSearch and Epic CTC platforms were analyzed for correlation to NEPC mCRPC status. Of note, 7/13 NEPC and atypical mCRPC patients had CellSearchⓇ CTC counts ≥5 CTC/7.5 mL (range 0-384), while 5/7 mCRPC patients had CellSearchⓇ CTC counts >5 CTCs/7.5mL. In contrast 17/17 NEPC and atypical mCRPC patients had CTC counts ≥5 CTC/7.5mL using the Epic platform. Further molecular and morphological characterization of the detected CTCs revealed that the heterogeneity of cytokeratin (CK) and AR expression also increased, with a far greater proportion of CK-negative CTCs, between NEPC and CRPC patients. With the exception of a single patient, the proportion of AR-negative CTCs increased in NEPC patients as well, consistent with NEPC disease progression and resistance to AR targeted therapies. Although CRPC patients were predominantly AR-positive, minor subpopulations of low AR and AR-negative CTCs with NEPC-like morphology could be detected. CTC clusters were observed in both NEPC and CRPC patients although at similar frequencies. Differences in heterogeneous subpopulations of CK+ and AR+ CTCs observed between NEPC and CRPC patients are shown in Figure 7.

Based on the observed differences in patient CTCs between groups, CTC characteristics specific to NEPC patient populations were identified. Kernel Density analysis (KDE) of the patient groups' CTCs in aggregate, identified significant differences in CK, AR and morphological characteristics when compared to CRPC. Specifically, the frequency of CTCs not expressing epithelial markers (CK-negative and dim CK), with smaller less circular cells, larger nuclear/cytoplasmic ratios and a higher frequency of more prominent macronucleoli were greater in NEPC CTCs. Differences in these characteristics indicate NEPC patients having a higher frequency of CTCs with characteristics consistent with mesenchymal stem cell like characteristics.

Identification of NEPC CTC. To demonstrate the diagnostic potential, the observed differences in CTC characteristics between patient classes were used to train a Random Forest classifier to identify NEPC vs. CRPC CTCs in a patient blood sample. Atypical CRPC patients were excluded from classifier training and cross-validation. Results from leave-one-out cross-validation of NEPC and CRPC samples are shown in Figures 7 and 8, where the output from the classifier is a p(NEPC+) value and a p(NEPC-) value for each CTC, corresponding to the estimated probability of the cell's class membership as NEPC+ and NEPC-.

From the density curve in Figure 9A, the samples from patients diagnosed with NEPC demonstrated a spike in the curves near the high end of the p(NEPC+) spectrum, with many curves peaking near a p(NEPC+) score of 95%. In Figure 9B, the number of CTCs/mL with p(NEPC+) scores greater than or equal to 95% are presented in a bar chart for each patient sample, where each column is colored by the actual clinical diagnosis that the classifier is trying to predict.

Obtaining positive signals at the CTC level from samples that the classifier does not encounter during training demonstrates the classifier's ability to detect NEPC from CRPC in a robust manner that mitigates the risk of over-fitting. These conditions simulate the environment that the classifier would face in practice, in the sense that any future blood sample sent in for NEPC analysis is presented to the algorithm as a series of CTCs that it has not encountered during training, which the classifier will then estimate the probability of class membership for each CTC from the new sample.

Atypical CRPC. The clinical significance of patients with adenocarcinoma CRPC that develop progressive disease in the setting of low serum PSA <1ng/ml, visceral metastases in the absence of PSA progression, or elevated serum chromogranin is not established. These tumors are less androgen responsive and are transitioning towards an AR negative/low or NEPC phenotype and demonstrate intratumoral heterogeneity with both adenocarcinoma and NEPC present within or between metastases. We applied the NEPC classification model to the 5 atypical CRPC patients and found that atypical CRPC is associated with an increase in heterogeneity of CRPC cells and a higher burden of NEPC related cells compared to CRPC patients (Figure 10, Figure 17).

Patient Case Studies. Atypical CRPC patient 6, for example, harbored CTCs of various morphologies with a predominance of NEPC CTCs (Figure 11A). Patient 6 is a 64 year old patient who presented with metastatic hormone naive prostate cancer, developed clinical progression within 6 months on primary hormonal therapy, was not responsive to subsequent abiraterone, radium-223, or docetaxel, and developed progressive bone metastases and new extensive liver and adrenal metastases in the setting of a stable PSA. Despite his bone biopsy showing adenocarcinoma without neuroendocrine features (Figure 11A), his clinical history supported AR independence. One potential explanation is that metastatic biopsy did not capture NEPC that may have been present at other metastatic sites.

Another example of how CTCs can be used to understand tumor heterogeneity is illustrated in the case of Patient 12. Patient 12 is a 68 year old gentleman with mCRPC who had a bone biopsy at the time of castration resistance for research which showed prostate adenocarcinoma. He was treated with abiraterone and prednisone. Despite PSA stability, follow-up imaging at 3 months on abiraterone revealed new liver and lung metastases and his serum chromogranin was markedly elevated serum chromogranin at 17,340 (ULN 95). Similar to Patient 6, PSA was stable despite visceral progression and Epic CTCs showed heterogeneous CTC populations including both NEPC and CRPC cells (Figure 11B), which might suggest intra-patient heterogeneity. In the case of patient 12, liver biopsy was performed and confirmed NEPC (small cell carcinoma) (Figure 11B). These cases support CTCs as potentially useful in capturing tumor heterogeneity that could not be assessed on one metastatic biopsy and warrants further investigation.

Histologic and molecular subtyping of cancer influences clinical decision making, and tissue confirmation is typically required at cancer diagnosis before treatment recommendations are offered. Prostate cancer is the most common cancer in men in the United States, and in nearly all cases diagnostic biopsies reveal adenocarcinoma. Prostate adenocarcinomas are characterized by AR expression and activation, and therefore hormonal therapies targeting the AR are the mainstay of systemic therapy (Chang et al., (2014) Nat Rev Clin Oncol 11: 308-323) Small cell neuroendocrine carcinoma of the prostate is a rare histologic subtype at diagnosis, representing <1% of all new prostate cancer diagnoses. However, in a subset of patients with metastatic prostate adenocarcinoma treated with AR targeted therapies, prostate adenocarcinomas can develop histologic transformation towards a predominantly neuroendocrine carcinoma as a mechanism of acquired resistance (Aggarwal et al. (2014) J Natl Compr Canc Netw 12: 719-726; Tagawa ST (2014) J Clin Oncol 32: 3360-3364; Beltran et al. (2012) J Clin Oncol 30: e386-389; Beltran et al. (2014) Clin Cancer Res 20: 2846-2850; Aparicio and Tzelepi (2014) Neuroendocrine (Small-Cell) Carcinomas: Why They Teach Us Essential Lessons About Prostate Cancer. Oncology (Williston Park)). NEPC phenotype is associated with rapid clinical progression, frequent visceral metastases, and low or absent AR expression on metastatic tumor biopsy. In this setting, patients are often offered platinum based chemotherapy with regimens similar to small cell neuroendocrine carcinoma of the lung (Aggarwal et al. (2014) J Natl Compr Canc Netw 12: 719-726; Santoni et al. (2014) Biochim Biophys Acta 1846: 630-637; Aparicio et al. (2013) Clin Cancer Res 19: 3621-3630). Therefore, identification of advanced prostate cancer patients that have acquired NEPC resistance has clinical implications.

However, the diagnosis of NEPC can be complex as there is a spectrum of morphologies seen in advanced prostate cancer with AR positive adenocarcinoma and pure AR negative small cell carcinoma representing the extreme phenotypes. Often times, metastatic biopsies reveal mixed features with both adenocarcinoma and neuroendocrine carcinoma observed and/or variable AR or neuroendocrine marker protein expression. The clinical significance of mixed tumors is less clear and treatment decisions are often individualized based on a combination of pathologic and clinical features. Furthermore, for patients with atypical clinical presentation such as rapid radiographic progression in setting of a low or modestly elevated PSA or elevated serum chromogranin, platinum-based therapies are sometimes considered even in the absence of neuroendocrine morphology on biopsy. Another challenge in the diagnosis of NEPC is that metastatic biopsies are not always feasible for patients suffering from advanced prostate cancer, may carry additional risks for the patient including complications from biopsy procedure or delay of initiation of appropriate systemic therapy for NEPC, and does not always capture disease heterogeneity. Therefore, a noninvasive marker to detect NEPC progression and simultaneously capture intratumoral heterogeneity is an unmet clinical need.

Morphologically, Epic CTCs from patients with NEPC were smaller in size and demonstrated abnormal nuclear and cytoplasmic features. Prior evidence suggests that prostate cancer tumor cells may undergo phenotypic changes associated with EMT during metastatic transit. Armstrong et al. (2011) Mol Cancer Res 9: 997-1007. Additionally, in cells that express both epithelial and mesenchymal markers, there may be an unknown number of CTCs that are EpCAM negative. Armstrong et al. (2011) Mol Cancer Res 9: 997-1007. Studies have suggested a link between loss of epithelial markers, gain of mesenchymal markers, and the induction of signaling pathways that promote survival and androgen-receptor independent growth (Zhau et al. (2008) Clin Exp Metastasis 25: 601-610; Arora et al. (2013) Cell 155: 1309-1322; Tanaka et al. (2010) Nat Med 16: 1414-1420) suggesting a mechanism of resistance to standard androgen deprivation therapy. The broad CTC heterogeneity of the atypical CRPC patients confirms the intra-tumoral heterogeneity with associated NEPC phenotypes. The demonstrated utility of the Epic platform to identify and characterize NEPC CTCs to monitor CRPC progressing patients for NEPC phenotypes particularly in patients with emerging atypical CRPC clinical characteristic.

As demonstrated herein, the Epic CTC Platform is capable of detecting CTCs from patients with NEPC like characteristics. The correlation of AuroraK status in CTCs to primary tumor demonstrates the utility of CTC characterization for the identification of NEPC disease progression using emerging molecular or genomic markers. NEPC CTCs have unique morphology, protein chemistry and genomics from CRPC CTCs and thus detection of them provides utility for early diagnosis of NEPC-associated acquired resistance. The results presented in this example confirm the feasibility of analyzing CTCs using the Epic platform, optionally in combination or in lieu of biopsies, prior to therapeutic decision points and throughout the course of treatment for real time monitoring of NEPC disease progression. With current diagnosis of NEPC being invasive and very difficult, the enablement of a liquid biopsy to accurately diagnosis NEPC provides an opportunity to better manage metastatic prostate cancer patients. Early detection and diagnosis of androgen insensitive NEPC improves therapeutic offerings, eventually leading to improvement of survival and health economics.

## Claims

1. A method for detecting neuroendocrine prostate cancer (NEPC) in a patient afflicted with prostate cancer comprising
(a) performing a direct analysis comprising immunofluorescent staining and morphological characterization of nucleated cells in a blood sample obtained from the patient to detect circulating tumor cells (CTC), wherein the direct analysis means the detection of CTCs in the context of all surrounding nucleated cells present in the sample as opposed to after enrichment of the sample for CTCs prior to detection; and
(b) determining presence or absence of a CTC subpopulation associated with NEPC comprising detecting a measurable feature of each biomarker characterizing the CTC subpopulation associated with NEPC in a panel of morphological and protein biomarkers,
wherein the presence of the CTC subpopulation associated with NEPC is indicative of NEPC,
wherein the biomarkers comprise small size, absence of Androgen Receptor (AR-), absence of cytokeratin (CK-) expression, and presence of nucleoli (nucleoli+),
wherein the small size is a cell size that is smaller than that of traditional CTCs, and
wherein the traditional CTCs are **characterized by** DAPI+CK+CD45- and are morphologically distinct from surrounding white blood cells (WBCs).

2. The method of claim 1, wherein the prostate cancer is metastatic castration resistant prostate cancer (mCRPC).

3. The method of claim 1, further comprising an initial step of depositing the nucleated cells as a monolayer onto a slide.

4. The method of claim 1, wherein the direct analysis comprises fluorescent scanning microscopy.

5. The method of claim 4, wherein the microscopy provides a field of view comprising CTCs and at least 200 surrounding WBCs.

6. The method of claim 1, wherein the CTCs comprise distinct morphological characteristics compared to surrounding nucleated cells.

7. The method of claim 6, wherein the morphological characteristics comprise one or more of the group consisting of nucleus size, nucleus shape, presence of holes in nucleus, cell size, cell shape and nuclear to cytoplasmic ratio, nuclear detail, nuclear contour, presence or absence of nucleoli, quality of cytoplasm and quantity of cytoplasm.

8. The method of claim 1, wherein the detection of CTCs further comprises comparing intensity of pan CK fluorescent staining to surrounding nucleated cells.

9. The method of claim 1, further comprising an initial step of obtaining a WBC count for the blood sample.

10. The method of claim 1, further comprising an initial step of lysing erythrocytes in the blood sample.

11. The method of claim 1, wherein the immunofluorescent staining of nucleated cells to detect CTCs comprises pan CK, cluster of differentiation (CD) 45, and diamidino-2-phenylindole (DAPI).

12. The method of claim 1, wherein determining the presence of a CTC subpopulation associated with NEPC in step (b) comprises analysis of the CTCs detected in step (a) at the single cell level.

## Patentansprüche

1. Verfahren zum Nachweisen von neuroendokrinem Prostatakrebs (NEPC) bei einem Patienten, der an Prostatakrebs leidet, umfassend
(a) Durchführen einer direkten Analyse, umfassend Immunfluoreszenzfärbung und morphologische Charakterisierung von kernhaltigen Zellen in einer von dem Patienten erhaltenen Blutprobe, um zirkulierende Tumorzellen (CTC) nachzuweisen, wobei die direkte Analyse den Nachweis von CTCs im Kontext aller umgebenden kernhaltigen Zellen, die in der Probe vorhanden sind, bedeutet, im Gegensatz zu nach Anreicherung der Probe auf CTCs vor dem Nachweis; und
(b) Bestimmen des Vorhandensein oder Fehlens einer mit NEPC assoziierten CTC-Subpopulation, umfassend das Nachweisen eines messbaren Merkmals jedes Biomarkers, der die mit NEPC assoziierte CTC-Subpopulation in einem Panel von morphologischen und Protein-Biomarkern charakterisiert,
wobei das Vorhandensein der mit NEPC assoziierten CTC-Subpopulation indikativ für NEPC ist,
wobei die Biomarker kleine Größe, Fehlen von Androgenrezeptor (AR-), Fehlen von Cytokeratin (CK-) Expression und Vorhandensein von Nukleoli (Nukleoli+) umfassen,
wobei die kleine Größe eine Zellgröße ist, die kleiner ist als die von traditionellen CTCs, und
wobei die traditionellen CTCs durch DAPI+CK+CD45- charakterisiert sind und sich morphologisch von den umgebenden weißen Blutzellen (WBCs) unterscheiden.

2. Verfahren nach Anspruch 1, wobei der Prostatakrebs ein metastasierender kastrationsresistenter Prostatakrebs (mCRPC) ist.

3. Verfahren nach Anspruch 1, das ferner einen anfänglichen Schritt des Aufbringens der kernhaltigen Zellen als Monolayer auf einen Objektträger umfasst.

4. Verfahren nach Anspruch 1, wobei die direkte Analyse Fluoreszenzraster-mikroskopie umfasst.

5. Verfahren nach Anspruch 4, wobei die Mikroskopie ein Sichtfeld bereitstellt, das CTCs und mindestens 200 umgebende WBCs umfasst.

6. Verfahren nach Anspruch 1, wobei die CTCs im Vergleich zu umgebenden kernhaltigen Zellen unterschiedliche morphologische Merkmale umfassen.

7. Verfahren nach Anspruch 6, wobei die morphologischen Merkmale eines oder mehrere aus der Gruppe umfassen, die aus Kerngröße, Kernform, Vorhandensein von Löchern im Kern, Zellgröße, Zellform und Kern zu Zytoplasma-Verhältnis, Kerndetail, Kernkontur, Vorhandensein oder Fehlen von Nukleoli, Qualität des Zytoplasmas und Quantität des Zytoplasmas besteht.

8. Verfahren nach Anspruch 1, wobei der Nachweis von CTCs ferner das Vergleichen der Intensität der pan-CK-Fluoreszenzfärbung mit umgebenden kernhaltigen Zellen umfasst.

9. Verfahren nach Anspruch 1, das ferner einen anfänglichen Schritt des Erhaltens einer WBC-Zahl für die Blutprobe umfasst.

10. Verfahren nach Anspruch 1, das ferner einen anfänglichen Schritt des Lysierens von Erythrozyten in der Blutprobe umfasst.

11. Verfahren nach Anspruch 1, wobei die Immunfluoreszenzfärbung von kernhaltigen Zellen zum Nachweisen von CTCs pan CK, Cluster of Differentiation (CD) 45 und Diamidin-2-phenylindol (DAPI) umfasst.

12. Verfahren nach Anspruch 1, wobei das Bestimmen des Vorhandenseins einer mit NEPC assoziierten CTC-Subpopulation in Schritt (b) die Analyse der in Schritt (a) detektierten CTCs auf Einzelzellebene umfasst.

## Revendications

1. Procédé de détection d'un cancer neuroendocrinien de la prostate (NEPC) chez un patient atteint d'un cancer de la prostate comprenant
(a) la réalisation d'une analyse directe comprenant la coloration immunofluorescente et la caractérisation morphologique de cellules nucléées dans un échantillon de sang prélevé chez le patient pour détecter des cellules tumorales circulantes (CTC), dans lequel l'analyse directe signifie la détection des CTC dans le cadre de toutes les cellules nucléées environnantes présentes dans l'échantillon par opposition à la période suivant l'enrichissement de l'échantillon de CTC avant la détection ; et
(b) la détermination de la présence ou de l'absence d'une sous-population de CTC associée au NEPC comprenant la détection d'une caractéristique mesurable de chaque biomarqueur caractérisant la sous-population de CTC associée au NEPC dans un panel de biomarqueurs morphologiques et protéiques,
dans lequel la présence de la sous-population de CTC associée au NEPC indique un NEPC,
dans lequel les biomarqueurs comprenant une taille réduite, l'absence du récepteur des androgènes (AR-), l'absence de l'expression des cytokératines (CK-) et la présence de nucléoles (nucléoles +),
dans lequel la taille réduite est une taille cellulaire qui est inférieure à celle des CTC habituelles, et
dans lequel les CTC habituelles sont **caractérisées par** DAPI+CK+CD45- et sont morphologiquement distinctes des globules blancs (WBC) environnants.

2. Procédé selon la revendication 1, dans lequel le cancer de la prostate est un cancer de la prostate métastatique résistant à la castration (mCRPC).

3. Procédé selon la revendication 1, comprenant en outre une étape initiale consistant à déposer les cellules nucléées en tant que monocouche sur une lame.

4. Procédé selon la revendication 1, dans lequel l'analyse directe comprend une microscopie à balayage par fluorescence.

5. Procédé selon la revendication 4, dans lequel la microscopie fournit un champ de vue comprenant des CTC et au moins 200 WBC environnants.

6. Procédé selon la revendication 1, dans lequel les CTC comprennent des caractéristiques morphologiques distinctes par rapport aux cellules nucléées environnantes.

7. Procédé selon la revendication 6, dans lequel les caractéristiques morphologiques comprennent un ou plusieurs parmi le groupe consistant en la taille du noyau, la forme du noyau, la présence de trous dans le noyau, la taille cellulaire, la forme cellulaire et le rapport nucléo-cytoplasmique, les détails nucléaires, les contours nucléaires, la présence ou l'absence de nucléoles, la qualité du cytoplasme et la quantité de cytoplasme.

8. Procédé selon la revendication 1, dans lequel la détection de CTC comprend en outre la comparaison de l'intensité de la coloration fluorescente de pan CK aux cellules nucléées environnantes.

9. Procédé selon la revendication 1, comprenant en outre une étape initiale consistant à obtenir une numération de WBC pour l'échantillon de sang.

10. Procédé selon la revendication 1, comprenant en outre une étape initiale consistant à lyser des érythrocytes dans l'échantillon de sang.

11. Procédé selon la revendication 1, dans lequel la coloration immunofluorescente de cellules nucléées pour détecter des CTC comprend les pan-CK, un amas de différenciation (CD) 45 et le diamidino-2-phénylindole (DAPI).

12. Procédé selon la revendication 1, dans lequel la détermination de la présence d'une sous-population de CTC associée au NEPC à l'étape (b) comprend l'analyse des CTC détectées à l'étape (a) au niveau unicellulaire.
